# EUROPÄISCHE PATENTSCHRIFT

(11) **EP 2 550 038 B1**
(45) Veröffentlichungstag und Bekanntmachung des Hinweises auf die Patenterteilung: **23.07.2014**
(21) Anmeldenummer: 11716474.9
(22) Anmeldetag: 23.03.2011
(51) Int. Cl.: A61M 1/36

(54) **VORRICHTUNG ZUM DETEKTIEREN VON FEUCHTIGKEIT ZUR VERWENDUNG MIT EINER VORRICHTUNG ZUR ÜBERWACHUNG EINES ZUGANGS ZU EINEM PATIENTEN**
MOISTURE DETECTION DEVICE FOR USE WITH A DEVICE FOR MONITORING AN ACCESS TO A PATIENT
DISPOSITIF POUR DÉTECTER L'HUMIDITÉ, POUR UTILISATION AVEC UN DISPOSITIF POUR LA SURVEILLANCE D'UN ACCÈS À UN PATIENT

(30) Priorität: 23.03.2010 DE 102010012545
(43) Veröffentlichungstag der Anmeldung: 30.01.2013
(73) Patentinhaber: Fresenius Medical Care Deutschland GmbH, 61352 Bad Homburg v.d.H. (DE)
(72) Erfinder: HEPPE, John, 66606 St. Wendel (DE)
(74) Vertreter: Oppermann, Frank
(86) Internationale Anmeldenummer: PCT/EP2011/001435
(87) Internationale Veröffentlichungsnummer: WO 2011/116943

(56) Entgegenhaltungen:
- WO-A1-2004/004615
- WO-A1-2006/008866
- WO-A2-2009/075592
- US-A- 5 790 036
- US-A1- 2002 198 483
- US-A1- 2009 322 543

## Beschreibung

Die Erfindung bezieht sich auf eine Vorrichtung zum Detektieren von Feuchtigkeit zur Verwendung mit einer Vorrichtung zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung, bei der Blut eines Patienten über eine arterielle Schlauchleitung, die eine arterielle Kanüle aufweist, von dem Patienten abgeführt wird, und über eine venöse Schlauchleitung, die eine venöse Punktionskanüle aufweist, dem Patienten zugeführt wird. Darüber hinaus betrifft die Erfindung eine Vorrichtung zur Überwachung eines Zugangs zu einem Patienten, die über eine Vorrichtung zum Detektieren von Feuchtigkeit verfügt. Des weiteren betrifft die Erfindung eine Blutbehandlungsvorrichtung mit einem extrakorporalen Blutkreislauf, der eine arterielle Schlauchleitung mit einer arteriellen Kanüle und eine venöse Schlauchleitung mit einer venösen Kanüle aufweist, wobei die extrakorporale Blutbehandlungsvorrichtung über eine Vorrichtung zur Überwachung des arteriellen und/oder venösen Gefäßzugangs verfügt. Die Erfindung betrifft auch ein Verfahren zur Herstellung von Vorrichtungen zum Detektieren von Feuchtigkeit zum Anschluss an eine Vorrichtung zur Überwachung eines Patientenzugangs.

Auf dem Gebiet der Medizintechnik sind verschiedene Einrichtungen bekannt, mit denen über eine Schlauchleitung Patienten Flüssigkeiten entnommen oder Flüssigkeiten den Patienten zugeführt werden können. Dabei erfolgt der Zugang zu den Patienten im Allgemeinen mit einem Katheter zum Einführen in Körperorgane oder einer Kanüle zum Punktieren von Gefäßen. Während der Untersuchung oder Behandlung ist ein ordnungsgemäßer Zugang zu dem Patienten sicher zu stellen. Daher ist es erforderlich, den Patientenzugang zu überwachen.

Einen ordnungsgemäßen Zugang zu dem Patienten setzen insbesondere auch die extrakorporalen Blutbehandlungsvorrichtungen voraus, die über einen extrakorporalen Blutkreislauf verfügen. Zu den bekannten extrakorporalen Blutbehandlungsvorrichtungen zählen beispielsweise Dialysevorrichtungen und Zellseparatoren, die einen Zugang zu dem Gefäßsystem des Patienten erforderlich machen. Bei der extrakorporalen Blutbehandlung wird dem Patienten über eine arterielle Schlauchleitung mit einer arteriellen Punktionskanüle Blut entnommen, das dem Patienten über eine venöse Schlauchleitung mit einer venösen Punktionskanüle wieder zugeführt wird.

Trotz regelmäßiger Überwachung des Gefäßzugangs durch das Krankenhauspersonal besteht grundsätzlich die Gefahr, dass die Punktionskanüle unbemerkt aus dem Blutgefäß des Patienten herausrutscht. Die Gefahr des unbemerkten Herausrutschens der Punktionskanüle besteht auch bei der Heimdialyse. Zur Überwachung des Gefäßzugangs sind verschiedene Vorrichtungen unterschiedlicher Ausbildung bekannt. Die bekannten Überwachungsvorrichtungen greifen im Allgemeinen auf die standardmäßig in den Blutbehandlungsvorrichtungen vorhandenen Sicherheitsvorrichtungen zurück, die bei einem nicht ordnungsgemäßen Gefäßzugang eine sofortige Unterbrechung des extrakorporalen Blutkreislaufs auslösen.

Es sind Vorrichtungen zur Überwachung eines Gefäßzugangs bekannt, die über eine Vorrichtung zum Detektieren von Feuchtigkeit verfügen, um an der Punktionsstelle das Austreten von Blut erkennen zu können. Die bekannten Vorrichtungen zum Detektieren von Feuchtigkeit, die bei den bekannten Überwachungsvorrichtungen für den Patientenzugang Verwendung finden, sind als auf die Punktionsstelle aufzulegendes Pad ausgebildet. Das Pad besteht aus einem saugfähigen Material, in das ein Feuchtigkeitssensor eingebettet ist.

Die WO 2006/008866 A1, US 2005/0038325 A1 und US 6,445,304 B1 beschreiben Vorrichtungen zum Detektieren von Feuchtigkeit aus einem saugfähigen Material, das auf die Haut aufgelegt wird. Die bekannten Pads zeichnen sich dadurch aus, dass der Feuchtigkeitssensor in das saugfähige Material eingebettet ist.

Aus der EP 1 537 264 B1 ist ein elektrisch leitfähiges Garn und ein Gewebe aus dem elektrisch leitfähigen Garn bekannt. Dieses Gewebe soll zur Abschirmung elektromagnetischer Felder oder zur Ableitung statischer Ladungen verwendet werden. Auch soll das Gewebe zur Datenübertragung und Stromversorgung dienen. Ein weiterer Verwendungszweck des elektrisch leitfähigen Garns wird in der Herstellung von Dehnungs- und Feuchtigkeitssensoren gesehen.

Ein Vlies aus synthetischen Fasern zur Abschirmung gegen elektromagnetische Störquellen wird in der DE 197 12 043 A1 beschrieben. Darüber hinaus sind aus mehreren Lagen bestehende Gewebe bekannt, bei denen einzelne Kreuzungspunkte von Kett- und Schussfäden elektrische Kontaktstellen bilden.

Die WO 2009/075592 A2 beschreibt eine Vorrichtung zum Detektieren von Feuchtigkeit in Form eines Gewebestreifens, auf dem oder in dem zwei parallele Leiterbahnen vorgesehen sind, zwischen denen der elektrische Widerstand gemessen wird. Die beiden Leiterbahnen werden durch leitfähige Garne gebildet, die nur in Längsrichtung des Gewebestreifens verlaufen. Elektrische Kontaktstellen zwischen sich kreuzenden Leiterbahnen sind nicht vorgesehen. Nachteilig ist, dass der Feuchtigkeitssensor aufgrund der Form der elektrisch leitenden Struktur nur eine relativ geringe Sensitivität hat.

Aus der WO 2004/004615 A1 ist ein Feuchtigkeitssensor bekannt, der insbesondere in einer Windel Verwendung finden soll. Der Feuchtigkeitssensor verfügt über zwei Elektroden, zwischen denen ein Dielektrikum aus einem für Flüssigkeit saugfähigen Material angeordnet ist. Bei einer Ausführungsform weist der Feuchtigkeitssensor zwei draht- oder fadenförmige Elektroden auf. Die beiden Elektroden sind mit dem Dielektrikum verwoben. Eine alternative Ausführungsform des Feuchtigkeitssensors verfügt über zwei nicht näher beschriebene Elektroden, die aber parallel zueinander verlaufen und mit nicht-leitfähigen Fäden oder Drähten verbunden sind. Die Elektroden mit den nicht-leitfähigen Fäden oder Drähten bilden einen Verbund mit einem Dielektrikum aus saugfähigem Material.

Der Erfindung liegt die Aufgabe zugrunde, eine in großen Stückzahlen kostengünstig herzustellende Vorrichtung zum Detektieren von Feuchtigkeit mit einer hohen Sensitivität zu schaffen, die einfach zu handhaben ist und einen hohen Tragekomfort bietet. Eine weitere Aufgabe der Erfindung liegt darin, eine Vorrichtung zur Überwachung eines Zugangs zu einem Patienten mit einer derartigen Vorrichtung zum Detektieren von Feuchtigkeit und eine extrakorporale Blutbehandlungsvorrichtung mit einer derartigen Vorrichtung zur Überwachung eines Patientenzugangs zu schaffen. Eine Aufgabe der Erfindung ist auch ein Verfahren zur kostengünstigen Herstellung von Vorrichtungen zum Detektieren von Feuchtigkeit in großen Stückzahlen anzugeben.

Die Lösung dieser Aufgaben erfolgt erfindungsgemäß mit den Merkmalen der unabhängigen Patentansprüche. Vorteilhafte Ausführungsformen der Erfindung sind Gegenstand der Unteransprüche.

Die erfindungsgemäße Vorrichtung zum Detektieren von Feuchtigkeit ist zum Anschluss an eine Vorrichtung zur Überwachung eines Zugangs zu einem Patienten bestimmt. Die erfindungsgemäße Vorrichtung ist als ein auf die Haut des Patienten aufzulegendes Flächengebilde ausgebildet, das als Feuchtigkeitssensor eine elektrisch leitende Struktur aufweist, an der die Vorrichtung zur Überwachung des Patientenzugangs anschließbar ist.

Die erfindungsgemäße Vorrichtung zum Detektieren von Feuchtigkeit zeichnet sich dadurch aus, dass das auf die Haut des Patienten aufzulegende Flächengebilde ein Gewebe ist, das sowohl aus nicht-leitfähigen Kett- und Schussfäden als auch leitfähigen Kett- und Schussfäden ausgebildet ist. Die leitfähigen und nicht-leitfähigen Kett- und Schussfäden sind derart angeordnet, dass die elektrisch leitende Struktur geschaffen wird. Durch räumliche Trennung der Kett- und Schussfäden wird in dem Gewebe eine definierte elektrisch leitende Struktur erzeugt.

Aus der Verwendung eines Gewebes zur Herstellung der Vorrichtung zum Detektieren von Feuchtigkeit ergeben sich in der Praxis entscheidende Vorteile. Ein entscheidender Vorteil in der Verwendung von sowohl leitfähigen Kettfäden als auch leitfähigen Schussfäden liegt darin, dass eine elektrisch leitende Struktur ausgebildet werden kann, die in unterschiedliche Richtungen verlaufende Abschnitte aufweist. Mit einer derartigen Struktur kann ein Feuchtigkeitssensor geschaffen werden, der eine besonders hohe Sensitivität aufweist.

Das Gewebe verfügt über sämtliche Eigenschaften, durch die sich die auf die Haut des Patienten aufzulegende Vorrichtung zum Detektieren von Feuchtigkeit auszeichnen soll. Hierzu gehören neben der erforderlichen Biokompatibilität auch die hohe Luftdurchlässigkeit und Saugfähigkeit. Die als textiles Flächengebilde ausgebildete Vorrichtung zum Detektieren von Flüssigkeit ist weich und flexibel und angenehm auf der Haut zu tragen. Bei geeigneter Auswahl der Materialien für die Kett- und Schussfäden kann eine hohe Biokompatibilität erzielt werden. Da die elektrisch leitende Struktur Bestandteil des Gewebes ist, sind zusätzliche Materialien zur Schaffung einer elektrisch leitenden Struktur, die nicht über die erforderliche Biokompatibilität verfügen könnten, nicht erforderlich. In einem konventionellen Webprozess kann die Vorrichtung zum Detektieren von Feuchtigkeit in hohen Stückzahlen kostengünstig hergestellt werden.

Der Produktionsprozess der erfindungsgemäßen Vorrichtung kann mit einem hohen Automatisierungsgrad erfolgen. So kann eine große Anzahl von hochsensitiven Sensoren mit einer einzigen Webmaschine auf einer Gewebebahn kostengünstig hergestellt werden, wobei die einzelnen Sensoren innerhalb des Prozesses oder später von der Gewebebahn getrennt werden können. Beispielsweise kann eine Webmaschine auf einer bis zu 3.000 mm breiten Gewebebahn bis zu 2.000 Sensoren pro Stunde herstellen. Untersuchungen haben gezeigt, dass die mit dem erfindungsgemäßen Verfahren hergestellten Sensoren gegenüber Leiterbahnbrüchen weitgehend unempfindlich sind und eine hohe Biegewechselfestigkeit aufweisen.

Die Vorrichtung zum Detektieren von Feuchtigkeit kann in unterschiedlichen Formen ausgebildet sein. Sie kann nicht nur bei Blutbehandlungsvorrichtungen verwendet werden, die einen Gefäßzugang über eine Kanüle oder Nadel schaffen, sondern ist grundsätzlich auch zur Verwendung bei Kathetern zum Zu- und Abführen von Flüssigkeiten geeignet.

Bei einer bevorzugten Ausführungsform der Vorrichtung zum Detektieren von Feuchtigkeit weist die elektrisch leitende Struktur eine erste Leiterbahn und eine zweite Leiterbahn auf, wobei die Enden der beiden Leiterbahnen als Anschlusskontakte ausgebildet sind. Wenn der zwischen den beiden Leiterbahnen liegende Bereich des Gewebes mit Blut in Kontakt kommt, verändert sich der zwischen den beiden Anschlusskontakten gemessene elektrische Widerstand. Wenn ein Abschlusswiderstand vorhanden ist, wird zwischen den Anschlusskontakten ein elektrischer Widerstand gemessen, der einer Paralleschaltung von dem Abschlusswiderstand und dem elektrischen Widerstand zwischen den Leiterbahnen entspricht. Dabei wird davon ausgegangen, dass das Blut die benachbarten Leiterbahnen überbrückt.

Zur Erhöhung der Sensitivität des Feuchtesensors sind die beiden Leiterbahnen vorzugsweise in einer Mehrzahl von Abschnitten nebeneinander liegend angeordnet. Dabei wird die Sensitivität mit zunehmender Anzahl von nebeneinander liegend angeordneten Abschnitten erhöht. Vorzugsweise sollte der gesamte auf der Vorrichtung zum Detektieren von Feuchtigkeit zur Verfügung stehende Raum für den Feuchtigkeitssensor ausgenutzt werden.

Eine alternative Ausführungsform sieht nur eine, aber als geschlossene Leiterschleife ausgebildete Leiterbahn für die elektrisch leitende Struktur vor, deren Enden als Anschlusskontakte ausgebildet sind.

Diese Ausführungsform setzt voraus, dass die Leiterbahn einen definierten Widerstand hat. Die Sensitivität des Feuchtigkeitssensors wird mit zunehmender Anzahl der nebeneinander liegend angeordneten Abschnitte der geschlossenen Leiterschleife erhöht. Wenn sich ein genau definierter Widerstand nicht in dem Produktionsprozess einstellen lassen sollte, kann der Widerstand auch bei der Anwendung des Sensors initial gemessen und als Referenzwert verwandt werden. Der längenspezifische Widerstand eines leitfähigen Fadens kann beispielsweise 100 Ohm pro Meter mit einer Abweichung von +/- 10% betragen. Es sind aber auch andere Werte möglich.

Aus der Verwendung eines Gewebes für die Herstellung der Vorrichtung zum Detektieren von Feuchtigkeit ergibt sich, dass die elektrisch leitende Struktur aus einer Mehrzahl von in einer ersten Richtung verlaufenden und einer Mehrzahl von in einer zweiten Richtung verlaufenden elektrisch leitfähigen Abschnitten zusammengesetzt ist, wobei die erste und zweite Richtung orthogonal zueinander stehen. Es können also in dem Gewebe eine oder zwei mäanderförmig verlaufende oder schneckenförmige Leiterbahnen erzeugt werden.

Eine besonders bevorzugte Ausführungsform sieht vor, dass das textile Flächengebilde zumindest teilweise als ein Gewebe mit mehreren Lagen ausgebildet ist. Das mehrlagige Gewebe erlaubt in dem Webprozess die elektrische Kontaktierung bzw. Isolation der sich an den Verbindungspunkten kreuzenden elektrisch leitfähigen Kett- und Schussfäden in unterschiedlichen Ebenen. Dadurch kann eine besonders sichere Kontaktierung bzw. Isolation der Kett- und Schutzfäden an den Kreuzungspunkten erzielt werden.

Eine bevorzugte Ausführungsform sieht ein Gewebe,mit drei Lagen vor. Die drei Gewebelagen können sich an allen Stellen des Sensors befinden oder nur an einzelnen Stellen des Sensors vorgesehen sein.

In dem dreilagigen Gewebe können die elektrisch leitfähigen und elektrisch nicht leitfähigen Kett- und Schussfäden derart angeordnet sein, dass eine auf die Haut des Patienten aufzulegende Lage, die nicht elektrisch leitend ist, eine Lage, in der die in der ersten Richtung verlaufenden elektrisch leitfähigen Abschnitte der Leiterbahn liegen, und eine Lage, in der die in der zweiten Richtung verlaufenden elektrisch leitfähigen Abschnitte der Leiterbahn liegen, ausgebildet ist. Zur Schaffung von elektrischen Kontaktstellen wechseln die elektrisch leitfähigen Kettfäden im Bereich der Kreuzungspunkte von elektrisch leitfähigen Kett- und Schussfäden die Ebenen derart, dass die Kett- und Schussfäden an den Kreuzungspunkten kontaktieren. Isolationsstellen werden dadurch geschaffen, dass sich kreuzende elektrisch leitfähige Kett- und Schussfäden aufgrund eines partiellen Wechsels der Ebenen nicht berühren.

Die einzelnen Abschnitte einer Leiterbahn können grundsätzlich von nur einem elektrisch leitfähigen Kett- oder Schussfaden gebildet werden. Es können aber auch mehrere nebeneinander verlaufende elektrisch leitfähige Kett- oder Schussfäden die Leiterbahnabschnitte bilden. Dadurch wird eine höhere Redundanz gegen Reißen der Fäden erzielt.

Bei der Herstellung des Gewebes mit der elektrisch leitenden Struktur können Bereiche dadurch strukturiert werden, dass das textile Flächengebilde in definierten Teilbereichen ausgeschnitten wird, sodass ein Teil von nebeneinander verlaufenden elektrisch leitfähigen Kett- oder Schussfäden durchtrennt wird. Bevorzugte Ausführungsformen der Erfindung sehen im Wesentlichen ringförmige oder kreuzförmige Ausschnitte in dem textilen Flächengebilde vor. Die Ausschnitte können aber auch jede beliebige andere Form haben. Sie können innerhalb oder am Rand des Gewebes liegen. Dabei können die Ausschnitte nicht nur zur weiteren Strukturierung der elektrisch leitenden Struktur, sondern auch zur Durchführung der Kanüle oder zur Fixierung der Vorrichtung zum Detektieren von Feuchtigkeit dienen.

Eine alternative Ausführungsform der Vorrichtung zum Detektieren von Feuchtigkeit, bei der das textile Flächengebilde ein mehrlagiges Gewebe ist, sieht zwischen der Lage, in der die in der ersten Richtung verlaufenden elektrisch leitfähigen Abschnitte der Leiterbahn liegen und der Lage, in der die in der zweiten Richtung verlaufenden elektrisch leitfähigen Abschnitte der Leiterbahn liegen, eine weitere Lage vor, mit der die Kett- und Schussfäden in diesen beiden Ebenen elektrisch voneinander isoliert werden.

Das textile Flächengebilde kann unterschiedliche Größen haben. Es sollte einerseits eine ausreichende Größe haben, um die Punktionsstelle vollständig abzudecken, andererseits nicht so groß sein, dass die Punktion behindert wird. Bevorzugte Ausführungsformen sehen ein U-förmiges oder kreisförmiges textiles Flächengebilde vor. Das U-förmige Gewebe ermöglicht, die Vorrichtung zum Detektieren von Feuchtigkeit auch dann anlegen zu können, wenn die Kanüle bereits gelegt ist. Das kreisförmige Gewebe hat vorzugsweise einen zentralen Ausschnitt zum Durchführen der Kanüle.

Eine weitere besonders bevorzugte Ausführungsform sieht eine Lasche an dem textilen Flächengebilde vor, an der die Anschlusskontakte angeordnet sind.

Eine weitere besonders bevorzugte Ausführungsform sieht vor, dass das textile Flächengebilde einen Abschnitt mit einem Ausschnitt und einen Abschnitt mit einer Abdeckung für den Ausschnitt aufweist, wobei die elektrisch leitende Struktur derart ausgebildet ist, dass das textile Flächengebilde an der Oberseite für Feuchtigkeit sensitiv ist. Der Vorteil dieser Ausführungsform liegt darin, dass der Ausschnitt des textilen Flächengebildes, in dem die Kanüle zu liegen kommt, mit der Abdeckung abgedeckt werden kann. Hierzu wird der Abschnitt mit der Abdeckung einfach auf den Abschnitt mit dem Ausschnitt geklappt. Dann ist der Feuchtigkeitssensor auf beiden Seiten sensitiv.

Die erfindungsgemäße Vorrichtung zur Überwachung eines Zugangs zu einem Patienten, insbesondere zur Überwachung des Gefäßzugangs bei einer extrakorporalen Blutbehandlung, verfügt über die erfindungsgemäße Vorrichtung zum Detektieren von Feuchtigkeit. Die Überwachungsvorrichtung weist vorzugsweise eine an die Vorrichtung zum Detektieren von Feuchtigkeit anschließbare Auswerteinheit auf, die bei der Detektion von Feuchtigkeit einen akustischen und/oder optischen und/oder taktilen Alarm auslöst. Auch kann ein Steuersignal für einen Eingriff in die Steuerung der Einrichtung erzeugt werden, mit der über die Schlauchleitung eine Flüssigkeit dem Patienten zugeführt und/oder eine Flüssigkeit von dem Patienten abgeführt wird.

Die Überwachungsvorrichtung weist vorzugsweise einen Anschlussteil auf, an dem die Vorrichtung zum Detektieren von Feuchtigkeit angeschlossen wird, um eine elektrische Verbindung zwischen der Auswerteinheit der Überwachungsvorrichtung und dem Feuchtigkeitssensor der Vorrichtung zum Detektieren von Feuchtigkeit herzustellen. Der Anschlussteil der Überwachungsvorrichtung ist vorzugsweise über ein Verbindungskabel ausreichender Länge mit der Auswerteinheit elektrisch verbunden. Alternativ kann aber auch eine drahtlose Verbindung aufgebaut werden.

Bei der Ausführungsform der Vorrichtung zum Detektieren von Feuchtigkeit, die eine als geschlossene Leiterschleife ausgebildete Leiterbahn mit zwei Anschlusskontakten aufweist, ist ein Abschlusswiderstand nicht vorgesehen. Bei der Ausführungsform mit zwei Leiterbahnen werden zwei Enden der Leiterbahnen über einen Abschlusswiderstand miteinander verbunden und die anderen Enden der Leiterbahnen elektrisch mit der Auswerteinheit der Überwachungsvorrichtung verbunden. Der Abschlusswiderstand ermöglicht bei der Ausführungsform mit den beiden Leiterbahnen die Überprüfung der Vorrichtung zum Detektieren von Feuchtigkeit auf ihre Funktionsfähigkeit durch eine Widerstandsmessung zwischen den Anschlusskontakten. Bei funktionsfähigem Feuchtigkeitssensor wird zwischen den Anschlusskontakten ein Widerstand gemessen, der der Summe des Abschlusswiderstands und des Leiterbahnwiderstands entspricht.

Von besonderem Vorteil ist bei der Ausführungsform mit den beiden über einen Abschlusswiderstand verbundenen Leiterbahnen, wenn der Abschlusswiderstand nicht Bestandteil der Vorrichtung zum Detektieren von Feuchtigkeit, sondern Bestandteil der Überwachungsvorrichtung ist. Dies hat den Vorteil, dass ein Abschlusswiderstand auf dem Gewebe oder in dem Gewebe nicht vorgesehen werden muss. Weiterhin ist vorteilhaft, dass der Abschlusswiderstand nach dem Austausch der zum einmaligen Gebrauch bestimmten Vorrichtung zum Detektieren von Feuchtigkeit nicht verworfen wird. Von Vorteil ist auch, dass sich ein separater Abschlusswiderstand leichter reproduzieren lässt als ein Widerstand auf oder in dem Gewebe. Ein aufgedruckter Abschlusswiderstand beispielsweise unterliegt viel größeren Fertigungstoleranzen. Die Fertigungstoleranz von beispielsweise Minitaturwiderständen (SMD-Widerstände) hingegen kann kleiner als 1% von dem Widerstandssollwert sein. Vorteilhaft ist auch, dass sich der Widerstandswert eines separaten Abschlusswiderstands im Gegensatz zu einem bedruckten Widerstand aufgrund von Biegewechselbeanspruchungen während der Dialysebehandlung nicht ändern kann.

Da der Abschlusswiderstand nicht Bestandteil der Vorrichtung zum Detektieren von Feuchtigkeit ist, können konventionelle Widerstände verwendet werden, insbesondere Miniaturwiderstände (SMD-Widerstände), die kostengünstig sind und geringe Bauteiltoleranzen haben. Auch kann sich der Abschlusswiderstand nicht ändern, wenn die Vorrichtung zum Detektieren von Feuchtigkeit Flüssigkeit ausgesetzt ist. Ferner entfällt bei der Produktion der Vorrichtung zum Detektieren von Flüssigkeit ein weiterer Produktionsschritt. Bei der Produktion des gewebten Feuchtigkeitsensors werden auch keine Lösungsmittel, Pasten oder dergleichen benötigt, wodurch die Biokompatibilität erhöht wird.

Bei einer besonders bevorzugten Ausführungsform befindet sich der Abschlusswiderstand in dem Anschlussteil der Überwachungsvorrichtung. Der Anschlussteil weist bei einer besonders bevorzugten Ausführungsform vier Anschlusskontakte auf, wobei an dem ersten und zweiten Anschlusskontakt das Verbindungskabel zur Herstellung einer elektrischen Verbindung zwischen der Auswerteinheit der Überwachungsvorrichtung und der Vorrichtung zum Detektieren von Feuchtigkeit angeschlossen ist, und der dritte und vierte Anschlusskontakt über den Abschlusswiderstand elektrisch miteinander verbunden ist. Dabei ist die Reihenfolge, in der die Anschlusskontakte angeordnet sind, beliebig. Es kommt nur darauf an, dass an zwei Anschlusskontakte eine Stromquelle und an zwei Anschlusskontake ein Abschlusswiderstand angeschlossen werden kann.

Der Anschlussteil ist vorzugsweise als Klemmvorrichtung zum Verklemmen des textilen Flächengewebes der Vorrichtung zum Detektieren von Feuchtigkeit ausgebildet. Die Klemmvorrichtung weist vorzugsweise Mittel auf, mit denen die Vorrichtung zum Detektieren von Feuchtigkeit derart ausgerichtet und/oder fixiert wird, dass die Anschlusskontakte der Vorrichtung zum Detektieren von Feuchtigkeit den entsprechenden Anschlusskontakten des Anschlussteils der Überwachungsvorrichtung gegenüberliegen. Diese Mittel können als der Form der Vorrichtung zum Detektieren von Feuchtigkeit entsprechende Ausnehmungen oder der Form der Ausschnitte der Vorrichtung zum Detektieren von Feuchtigkeit entsprechende Ansätze ausgebildet sein. Die Fixierung kann durch Formschluss, Kraftschluss oder Reibschluss erfolgen. Als Mittel zum Fixieren können auch die Anschlusskontakte des Anschlussteils der Überwachungsvorrichtung selbst ausgebildet sein. Beispielsweise können die Anschlusskontakte in das Gewebe eindringende Dorne sein.

Die erfindungsgemäße Vorrichtung zur Überwachung eines Patientenzugangs kann eine separate Einheit bilden oder Bestandteil der Einrichtung sein, mit der dem Patienten eine Flüssigkeit zugeführt und/ oder von dem Patienten Flüssigkeit abgeführt wird, insbesondere Bestandteil der extrakorporalen Blutbehandlungsvorrichtung sein. Wenn die erfindungsgemäße Überwachungsvorrichtung Bestandteil der Blutbehandlungsvorrichtung ist, kann die Überwachungsvorrichtung von bestimmten Baugruppen oder Bauteilen Gebrauch machen, die in der Blutbehandlungsvorrichtung ohnehin vorhanden sind.

Die Seite der Vorrichtung zum Detektieren von Feuchtigkeit, die auf die Haut des Patienten aufgelegt wird, ist vorzugsweise mit einer vorzugsweise für Feuchtigkeit undurchlässigen Adhäsions- oder Klebschicht zur Fixierung der Vorrichtung zum Detektieren von Feuchtigkeit auf der Haut bedeckt. Vorzugsweise wird auf die Adhäsions- oder Klebschicht ein die Schicht abdeckendes Deckmaterial aufgebracht, das leicht von dem Trägermaterial abgezogen werden kann.

Bei der Herstellung der Vorrichtung zum Detektieren von Feuchtigkeit erweist sich als vorteilhaft, dass auf die gewebten Bahnen die Adhäsions- oder Klebschicht in dem Webprozess zusammen mit dem Deckmaterial kontinuierlich auf das textile Flächengebilde leicht aufgebracht werden kann. Die Vorrichtungen zum Detektieren von Feuchtigkeit liegen dann als Rollenware vor und brauchen nur voneinander getrennt zu werden. Vorzugsweise werden die einzelnen Vorrichtungen zum Detektieren von Feuchtigkeit direkt nach dem Aufbringen der Adhäsions- oder Klebschicht und des Deckmaterials in der gewünschten Form ausgeschnitten oder ausgestanzt.

Anstelle der Adhäsions- oder Klebeschicht kann auch eine vorzugsweise für Feuchtigkeit undurchlässige Klebefolie, beispielsweise eine PET-Folie, auf das Gewebe aufgebracht werden. Die Klebefolie hat einerseits den Vorteil, dass eine Barriere gegen Durchnässung des Gewebes mit Schweiß des Patienten geschaffen werden kann, andererseits eine unterschiedliche Klebkraft an der Ober- und Unterseite vorgesehen werden kann. Vorzugsweise weist die Folie an der der Haut des Patienten zugewandten Seite eine kleinere Klebkraft als der der Haut abgewandten und dem Gewebe zugewandten Seite auf.

Im Folgenden werden verschiedene Ausführungsbeispiele der Erfindung unter Bezugnahme auf die Zeichnungen näher erläutert.

Es zeigen:
- Fig. 1: die wesentlichen Komponenten einer Blutbehandlungsvorrichtung, die über eine Vorrichtung zur Überwachung des arteriellen und venösen Gefäßzugangs verfügt,
- Fig. 2: einen Schnitt durch das Gewebe,
- Fig. 3A: eine schematische Darstellung der ersten Gewebelage der Vorrichtung zum Detektieren von Feuchtigkeit,
- Fig. 3B: eine schematische Darstellung der zweiten Gewebelage der Vorrichtung zum Detektieren von Feuchtigkeit,
- Fig. 3C: eine schematische Darstellung der dritten Gewebelage der Vorrichtung zum Detektieren von Feuchtigkeit,
- Fig. 3D: eine schematische Darstellung der zweiten und dritten Gewebelage der Vorrichtung von Fig. 3B und Fig. 3C,
- Fig. 3E: eine schematische Darstellung der ersten Leiterbahn,
- Fig. 3F: eine schematische Darstellung der zweiten Leiterbahn,
- Fig. 3G: eine Darstellung der Leiterbahnen der zweiten und dritten Gewebelage,
- Fig. 3H: eine Darstellung der durchlaufenden elektrisch leitfähigen Kett- und Schussfäden mit den Leiterbahnen der zweiten und dritten Gewebelage,
- Fig. 4A: die erste Gewebelage einer zweiten Ausführungsform der Vorrichtung zum Detektieren von Feuchtigkeit mit einer weiteren isolierenden Gewebelage;
- Fig. 4B: die zweite Gewebelage der Vorrichtung zum Detektieren von Feuchtigkeit,
- Fig. 4C: die isolierende dritte Gewebelage,
- Fig. 4D: die vierte Gewebelage,
- Fig. 4E: eine Darstellung der elektrisch leitenfähigen Kett- und Schussfäden der zweiten und vierten Gewebelage,
- Fig. 4F: eine schematische Darstellung der ersten Leiterbahn,
- Fig. 4G: eine schematische Darstellung der zweiten Leiterbahn,
- Fig. 4H: eine Darstellung der Leiterbahnen der zweiten und vierten Gewebelage,
- Fig. 4I: eine Darstellung der Leiterbahnen der zweiten und vierten Gewebelage zusammen mit den durchlaufenden elektrisch leitenden Kett- und Schussfäden,
- Fig. 5A: die erste Gewebelage eines weiteren Ausführungsbeispiels der Vorrichtung zum Detektieren von Feuchtigkeit,
- Fig. 5B: die zweite Gewebelage,
- Fig. 5C: die dritte Gewebelage,
- Fig. 5D: eine Darstellung der leitenden Kett- und Schussfäden der zweiten und dritten Gewebelage,
- Fig. 5E: eine Darstellung der Leiterbahn der Vorrichtung zum Detektieren von Feuchtigkeit,
- Fig. 5F: eine Darstellung der Leiterbahn zusammen mit den durchlaufenden Kett- und Schussfäden,
- Fig. 5G: die Leiterbahn mit einem an der Oberseite isolierten Gewebebereich,
- Fig. 6: ein erstes Ausführungsbeispiel des Anschlussteils der Vorrichtung zum Überwachen eines Patientenzugangs,
- Fig. 7: ein zweites Ausführungsbeispiel des Anschlussteils der Überwachungsvorrichtung,
- Fig. 8: eine Darstellung der Verfahrensschritte zur Herstellung der Vorrichtung zum Detektieren von Feuchtigkeit,
- Fig. 9: eine weitere Ausführungsform der Vorrichtung zum Detektieren von Feuchtigkeit in schematischer Darstellung,
- Fig. 10: eine Matrix zur Veranschaulichung der Kreuzungspunkte der Kett- und Schussfäden der Vorrichtung zum Detektieren von Feuchtigkeit von Fig. 9,
- Fig. 11: ein elektrisches Ersatzschaltbild der Vorrichtung von Fig. 9,
- Fig. 12: eine Darstellung zur Veranschaulichung von sensitiven Bereichen der Vorrichtung von Fig. 9,
- Fig. 13: eine Darstellung zur Veranschaulichung von verschiedenen Schnitten durch die Vorrichtung von Fig. 9,
- Fig. 14A - Fig. 14E: eine Darstellung zur Veranschaulichung der Verknüpfungen zwischen Kett- und Schussfäden der Vorrichtung von Fig. 9 in den Schnittebenen von Fig. 13,
- Fig. 15: ein Ausführungsbeispiel der Lasche der Vorrichtung zum Detektieren von Feuchtigkeit,
- Fig. 16: ein weiteres Ausführungsbeispiel der Lasche der Vorrichtung zum Detektieren von Feuchtigkeit,
- Fig. 17A: ein weiteres Ausführungsbeispiel der Vorrichtung zum Detektieren von Feuchtigkeit in einer Seitenansicht,
- Fig. 17B: die Vorrichtung zum Detektieren von Feuchtigkeit von Fig. 17A in der Draufsicht zusammen mit einer Darstellung der einzelnen Lagen in einer Tabelle,
- Fig. 18: ein weiteres Ausführungsbeispiel der Vorrichtung zum Detektieren von Feuchtigkeit zusammen mit einer Darstellung der einzelnen Lagen in einer Tabelle,
- Fig. 19: ein weiteres Ausführungsbeispiel der Vorrichtung zum Detektieren von Feuchtigkeit zusammen mit einer Darstellung der einzelnen Lagen in einer Tabelle,
- Fig. 20: ein weiteres Ausführungsbeispiel der Vorrichtung zum Detektieren von Feuchtigkeit zusammen mit einer Darstellung der einzelnen Lagen in einer Tabelle,
- Fig. 21: ein weiteres Ausführungsbeispiel der Vorrichtung zum Detektieren von Feuchtigkeit zusammen mit einer Darstellung der einzelnen Lagen,
- Fig. 22: ein weiteres Ausführungsbeispiel der Vorrichtung zum Detektieren von Feuchtigkeit zusammen mit einer Darstellung der einzelnen Lagen und
- Fig. 23: ein weiteres Ausführungsbeispiel der Vorrichtung zum Detektieren von Feuchtigkeit zusammen mit einer Darstellung der einzelnen Lagen.

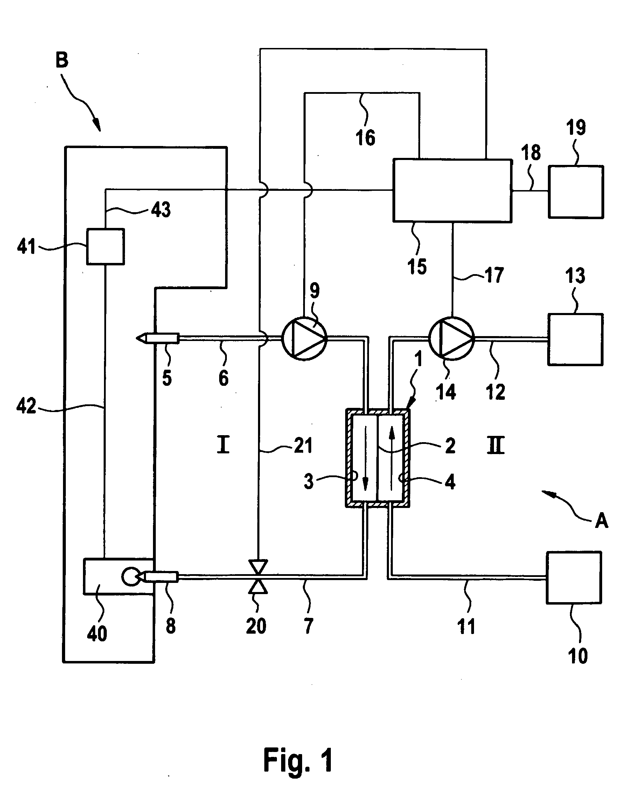

Fig. 1 zeigt die wesentlichen Komponenten einer Blutbehandlungsvorrichtung, insbesondere Hämodialysevorrichtung A, die über eine Vorrichtung B zur Überwachung des arteriellen und venösen Gefäßzugangs verfügt. Bei dem vorliegenden Ausführungsbeispiel ist die Überwachungsvorrichtung B Bestandteil der Hämodialysevorrichtung A. Zunächst wird die Dialysevorrichtung unter Bezugnahme auf Fig. 1 beschrieben.

Die Hämodialysevorrichtung A weist einen Dialysator 1 auf, der durch eine semipermeable Membran 2 in eine Blutkammer 3 und eine Dialysierflüssigkeitskammer 4 unterteilt ist. An der Fistel oder dem Shunt des Patienten ist mittels einer arteriellen Punktionskanüle 5 eine arterielle Schlauchleitung 6 angeschlossen, die zu dem Einlass der Blutkammer des Dialysators führt. Von dem Auslass der Blutkammer 3 des Dialysators 1 geht eine venöse Schlauchleitung 7 ab, die mittels einer venösen Punktionskanüle 8 an der Fistel oder dem Shunt des Patienten angeschlossen ist. In die arterielle Schlauchleitung 6 ist in eine Blutpumpe 9 geschaltet, die das Blut im extrakorporalen Blutkreislauf I fördert.

Der Dialysierflüssigkeitskreislauf II der Dialysevorrichtung A umfasst eine Dialyseflüssigkeitsquelle 10, an der eine Dialysierflüssigkeitszuführleitung 11 angeschlossen ist, die zu dem Einlass der Dialysierflüssigkeitskammer 4 des Dialysators führt. Von dem Auslass der Dialysierflüssigkeitskammer 4 des Dialysators 1 geht eine Dialysierflüssigkeitsabführleitung 12 ab, die zu einem Auslass 13 führt. In die Dialysierflüssigkeitsabführleitung 12 ist eine Dialysierflüssigkeitspumpe 14 geschaltet.

Die Steuerung der Dialysevorrichtung übernimmt eine zentrale Steuereinheit 15, die über Steuerleitungen 16, 17 die Blut- und Dialysierflüssigkeitspumpe 9, 14 ansteuert. Die zentrale Steuereinheit 15 ist über eine Datenleitung 18 mit einer Alarmeinheit 19 verbunden, die bei einem Störfall einen optischen und/oder akustischen und/oder taktilen Alarm gibt.

Stromab der Blutkammer 3 des Dialysators 1 befindet sich an der venösen Schlauchleitung 7 eine elektromagnetisch betätigbare Schlauchklemme 20, die über eine weitere Steuerleitung 21 von der zentralen Steuereinheit 15 geschlossen wird, wenn die venöse Punktionskanüle (Nadel) aus dem Gefäßzugang herausrutschen sollte und der Feuchtigkeitssensor mit Blut befeuchtet werden sollte. Darüber hinaus stoppt die Steuereinheit 15 nach dem Herausrutschen der Kanüle bei Befeuchtung des Sensors mit Blut die Blutpumpe 9.

Die Überwachungsvorrichtung B dient bei dem vorliegenden Ausführungsbeispiel zur Überwachung des venösen Gefäßzugangs. Die Überwachungsvorrichtung B verfügt über eine Vorrichtung 40 zur Detektion von Feuchtigkeit, die an der Punktionsstelle angeordnet wird. Diese Detektionsvorrichtung 40 ist in Fig. 1 nur schematisch dargestellt. Darüber hinaus verfügt die Überwachungsvorrichtung über eine Auswerteinheit 41, die über eine Verbindungsleitung 42 mit der Detektionsvorrichtung 40 elektrisch verbunden ist.

Über eine Datenleitung 43 ist die Auswerteinheit 41 mit der zentralen Steuereinheit 15 der Dialysevorrichtung A verbunden. Für den Fall, dass Blut aus der venösen Kanüle und/oder der Punktionsstelle austritt und den Feuchtigkeitssensor befeuchtet, erzeugt die Auswerteinheit 41 der Überwachungsvorrichtung B ein Steuersignal, das die zentrale Steuereinheit 15 über die Datenleitung 43 empfängt, die einen Eingriff in die Blutbehandlung vornimmt. Die Steuereinheit 15 stoppt die Blutpumpe 9 und schließt die Schlauchklemme 20. Darüber hinaus erzeugt die Steuereinheit ein Alarmsignal, sodass die Alarmeinheit 19 einen akustischen und/oder optischen und/oder taktilen Alarm gibt. Die Daten können zwischen der Überwachungsvorrichtung B und der Dialysevorrichtung A auch drahtlos übertragen werden.

Nachfolgend wird ein erstes Ausführungsbeispiel der auf die Haut des Patienten an der Punktionsstelle aufzulegenden Vorrichtung 40 zum Detektieren von Feuchtigkeit beschrieben. Die Detektionsvorrichtung 40 ist als ein auf die Haut des Patienten aufzulegendes Pad aus einem textilen Flächengebilde (Gewebe) ausgebildet. Bei dem ersten Ausführungsbeispiel ist das textile Flächengebilde 100 ein mehrlagiges Gewebe, das drei Lagen (Ebenen) aufweist.

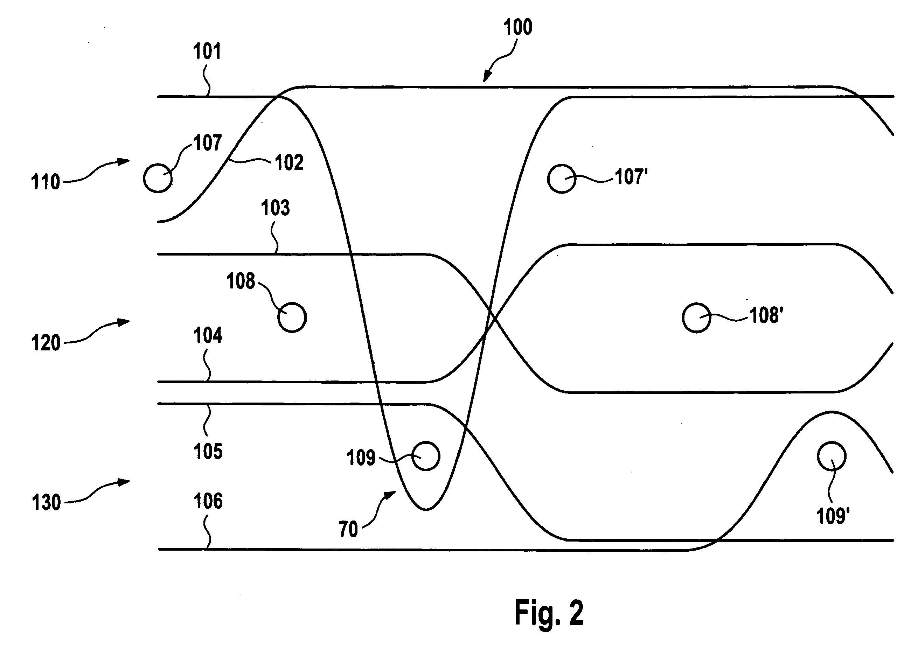

Fig. 2 zeigt einen Kettschnitt durch das dreilagige Gewebe 100. In Fig. 2 sind die von links nach rechts verlaufenden Kettfäden dargestellt. Der Kettschnitt zeigt insgesamt 6 Kettfäden 101 - 106. Die Anzahl der Lagen des Gewebes ist nach der Anzahl der Ebenen 110, 120, 130 definiert, in der die Schussfäden, 107, 108, 109; 107', 108', 109' liegen. Die in den drei Ebenen 110, 120, 130 im Wesentlichen rechtwinklig zu den Kettfäden liegenden Schussfäden 107, 108, 109; 107', 108', 109' sind durch Kreise gekennzeichnet. Die Herstellung eines dreilagigen Gewebes ist dem Fachmann bekannt. Beim Weben liegen die Schussfäden 107, 108, 109; 107', 108', 109' auf drei Ebenen 100, 110, 120. Die Kettfäden 101 - 106 werden auf drei Ebenen zugeführt. Aus den drei Kettfädenebenen heraus kann jeder einzelne Kettfaden jeweils angehoben oder abgesenkt werden, um das Durchschießen eines Schussfadens zu ermöglichen. Bei dem dreilagigen Gewebe werden in der Produktion aus ursprünglich 60 Fäden/cm auf einer Ebene 20 Fäden in einer oberen Ebene zugeführt, 20 Fäden in einer mittleren Ebene zugeführt und 20 Fäden in einer unteren Ebene zugeführt. Die Anzahl von 60 Fäden/cm stellt ein übliches Beispiel dar, kann jedoch auch davon abweichen.

Die Schussfäden 107, 108, 109; 107', 108', 109' müssen beim Webprozess nicht zwingend in übereinander liegenden Ebenen zugeführt werden, sondern die Lage eines Schussfadens in einer Ebene kann sich auch im Webprozess durch zurückschnellen" der angehobenen oder abgesenkten Kettfäden ergeben, die den Schussfaden dabei zwangsläufig in eine definierte Ebene ziehen. Die Ebenen sind stets als "gedachte" Lagen zu verstehen, die nicht "plan" sein müssen.

Bei dem vorliegenden Ausführungsbeispiel hat die Detektionsvorrichtung 40, die nachfolgend auch als Pad bezeichnet wird, die Form eines U. Das U-förmige Pad 40 weist einen mittleren Bereich 40A mit zwei Schenkeln 40B, 40C auf, die einen halbkreisförmigen Ausschnitt 40D seitlich umschließen. An den mittleren Abschnitt 40A ist eine den beiden Schenkeln 40B, 40C gegenüber liegende Lasche 40E angeformt.

Das Mehrlagengewebe besteht aus elektrisch leitfähigen und elektrisch nicht leitfähigen Kett- und Schussfäden (Monofilamente, Carbonfasern, versilbertes Polyamidgarn). Die elektrisch leitfähigen und elektrisch nicht leitfähigen Kett- und Schussfäden sind derart angeordnet, dass das Gewebe eine auf die Haut des Patienten aufzulegende untere Lage, eine mittlere Lage und eine der Haut des Patienten abgewandte obere Lage aufweist.

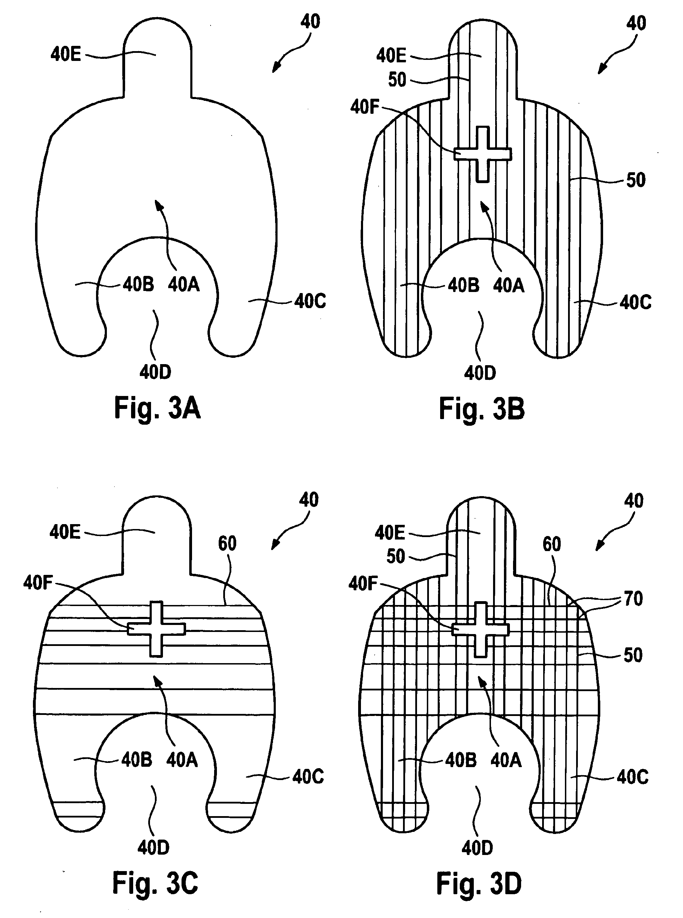

Fig. 3A zeigt die untere Lage des Gewebes. Die untere Gewebelage ist elektrisch nicht leitend. In dieser Ebene befinden sich keine elektrisch leitfähigen Kett- und Schussfäden. Auf die untere Lage kann aber auch verzichtet werden. Die elektrisch leitfähigen Kett- und Schussfäden befinden sich in der mittleren und oberen Ebene. In diesen beiden Ebenen bilden die leitfähigen und nicht leitfähigen Kett- und Schussfäden eine elektrisch leitfähige Struktur. Bei der elektrisch leitfähigen Struktur handelt es sich um zwei Leiterbahnen, die sich jeweils über das gesamte Pad erstrecken. Beide Leiterbahnen setzen sich aus jeweils rechtwinklig zueinander verlaufenden Abschnitten zusammen, wie nachfolgend erläutert wird.

Fig. 3B zeigt die mittlere Lage des Gewebes. Die elektrisch leitfähigen Kettfäden 50, die in der mittleren Ebene liegen, sind durch senkrechte Linien gekennzeichnet. Diese Kettfäden bilden die in einer ersten Richtung verlaufenden Abschnitte der beiden Leiterbahnen, wenn sie durch Schaffung geeigneter Kontakt- oder Isolationsstellen einer Leiterbahn "zugeordnet" werden.

Fig. 3C zeigt die obere Lage des Gewebes. Die elektrisch leitfähigen Schussfäden 60 sind durch horizontale Linien gekennzeichnet. Diese Schussfäden bilden die Abschnitte beider Leiterbahnen, die sich in der zu der ersten Richtung orthogonal verlaufenden zweiten Richtung erstrecken, wenn sie durch Schaffung geeigneter Kontakt- oder Isolationsstellen einer Leiterbahn "zugeordnet" werden.

In Fig. 3D sind die elektrisch leitfähigen Kett- und Schussfäden 50, 60 des Gewebes durch senkrechte und horizontale Linien gekennzeichnet. Es ergibt sich eine rasterförmige Struktur von elektrisch leitfähigen Fäden.

Die beiden Leiterbahnen 80, 90 werden in der mittleren und oberen Ebene des Gewebes dadurch gebildet, dass die elektrisch leitfähigen Kett- und Schussfäden 50, 60 an den Kreuzungspunkten 70 derart angeordnet werden, dass sie entweder elektrisch leitend miteinander verbunden sind oder elektrisch voneinander isoliert sind. Eine Kontaktstelle zwischen elektrisch leitfähigen Kett- und Schussfäden wird mittels eines partiellen Ebenenwechsels des Kettfadens während des Webprozesses erzielt, wie aus Fig. 2 ersichtlich ist.

Fig. 2 zeigt die in den drei Ebenen 110, 120, 130 liegenden Schussfäden 107, 108, 109; 107', 108', 109'. Durch den partiellen Wechsel des elektrisch leitfähigen Kettfadens 102 beispielsweise von der oberen Ebene 110 in die untere Ebene 130 wird eine elektrische Verbindung zwischen diesem Kettfaden 101 und demjenigen elektrisch leitenden Schussfaden 109 in der unteren Ebene hergestellt, der den Kettfaden 102 kreuzt. Ohne den partiellen Wechsel der Ebenen sind elektrisch leitfähige Kett- und Schussfäden voneinander isoliert. Beispielsweise ist der elektrisch leitfähige Kettfaden 102 nicht elektrisch mit dem elektrisch leitfähigen Schussfaden 109 verbunden, da der Kettfaden 102 im Bereich des Schussfadens 109 die Ebene partiell nicht wechselt.

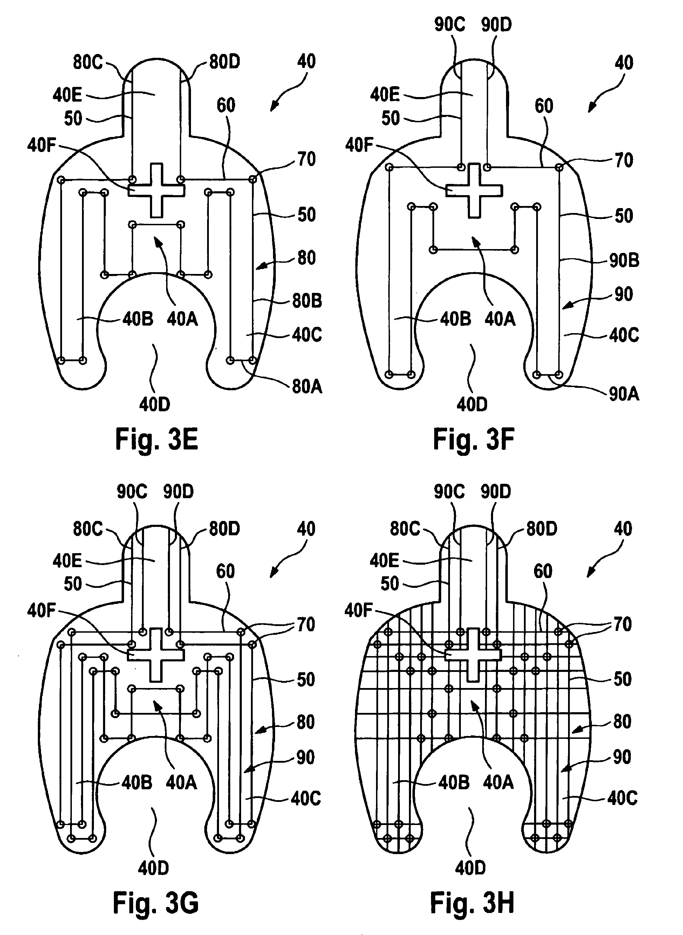

In Fig. 3E sind die elektrischen Kontaktstellen an den Kreuzungspunkten 70 zwischen den elektrisch leitfähigen Kettfäden 50 von Fig. 3B und den elektrisch leitfähigen Schussfäden 60 von Fig. 3C als Kreise dargestellt. Es ergibt sich als Leiterbahn 80 eine geschlossene Leiterschleife, die von der Lasche 40E über den mittleren Bereich 40A zu dem linken Schenkel 40B und von dem linken Schenkel 40B über den mittleren Bereich 40A zu dem rechten Schenkel 40C und von dem rechten Schenkel 40C über den mittleren Bereich 40A zurück zu der Lasche 40E des Pad 40 verläuft. Deutlich sind die orthogonal zueinander stehend geraden Abschnitte 80A, 80B der ersten Leiterbahn 80 zu erkennen. Die beiden Enden der Leiterbahn 41 bilden die beiden Anschlusskontakte 80C, 80D der ersten Leiterbahn 80. Beide Anschlusskontakte 80C, 80D liegen an der Außenseite der Lasche 40E.

Die zweite Leiterbahn 90 mit den senkrecht zueinander verlaufenden Abschnitten 90A, 90B ist in Fig. 3F dargestellt. Sie verläuft wieder von der Lasche 40E über den mittleren Bereich 40A zu dem linken Schenkel 40B und von dem linken Schenkel 40B über den mittleren Bereich 40A zu dem rechten Schenkel 40C und von dem rechten Schenkel 40C über den mittleren Bereich 40A zu der Lasche 40E des Pad 40. Die beiden Enden der zweiten Leiterbahn bilden ein zweites Paar von Anschlusskontakten 90C 90D, die an der Lasche 40E zwischen den Anschlusskontakten 80C, 80D der ersten Leiterbahn 80 angeordnet sind.

Fig. 3G zeigt die beiden Leiterbahnen 80, 90 zusammen mit den Kontaktstellen. Die einzelnen Abschnitte 80A, 80B, 90A, 90B beider Leiterbahnen 80, 90 sind derart angeordnet, dass sie im Wesentlichen parallel zueinander verlaufen.

Der besseren Übersichtlichkeit halber sind in den Figuren 3E bis 3G nur die Abschnitte der leitfähigen Kett- und Schussfäden eingezeichnet, die die Leiterbahnen bilden. Die Kett- und Schussfäden durchlaufen aber das Gewebe über die gesamte Breite und Länge.

Fig. 3H zeigt zur Veranschaulichung die elektrisch leitfähigen Kett- und Schussfäden 50, 60 beider Leiterbahnen 80, 90 über ihre gesamte Länge. Die sich kreuzenden Kett- und Schussfäden kontaktieren aber nur an den durch Kreise dargestellten Kontaktstellen.

Bei dem vorliegenden Ausführungsbeispiel werden die elektrisch leitfähigen Kett- und Schussfäden zum einen durch den halbkreisförmigen Ausschnitt 40D durchtrennt. Zum anderen werden die leitfähigen Kett- und Schussfäden durch einen weiteren Ausschnitt 40F getrennt, der sich in dem mittleren Abschnitt 40B des Pad 40 befindet. Bei dem vorliegenden Ausführungsbeispiel ist dieser Ausschnitt ein kreuzförmiger Ausschnitt 40F. Dieser Ausschnitt kann aber auch jede beliebige andere Form haben. Entscheidend ist, dass mit einem oder mehreren zusätzlichen Ausschnitten gezielt eine elektrische Struktur einer besonderen Ausbildung geschaffen werden kann, in der einzelne leitfähige Fäden getrennt sind.

Der inbesondere kreuzförmige Ausschnitt 40F dient zum Einen der gezielten, nachträglichen und dauerhaften Unterbrechung der leitfähigen Fäden im fertigen Gewebe, sodass im fertigen Produkt nur ein einziger Leiterbahnpfad verbleibt. Mit dem Ausschnitt 40F soll vermieden werden, dass redundante Leiterbahnpfade verbleiben. Zum Anderen kann der Ausschnitt 40F in Verbindung mit einem entsprechend ausgebildeten Ansatz der Fixierung der Anschlusskontakte durch Formschluss dienen.

Der halbkreisförmigen Ausschnitt 40D dient zum Durchführen der Punktionskanüle, wobei das Pad 40 auch dann auf die Haut des Patienten aufgelegt werden kann, wenn die Punktionskanüle bereits gelegt ist. Die mittlere Ausnehmung 40F kann zur Ausrichtung und/oder Fixierung des Pad in einem geeigneten Halte -order Klemmteil dienen, das aber in den Figuren nicht dargestellt ist.

Nachfolgend werden weitere Ausführungsbeispiele des Pad beschrieben, die sich aber von dem unter Bezugnahme auf die Fig. 3A - 3H beschriebenen Ausführungsbeispiel nur durch die Form des Pad sowie die elektrische Strukturierung unterscheiden. Sämtliche Ausführungsformen beruhen auf dem gleichen Grundprinzip, durch gezielte Schaffung von Kontaktstellen oder Isolationsstellen elektrisch leitfähige Kett- und Schussfäden in einem Gewebe aus elektrisch leitfähigen und elektrisch nicht leitfähigen Kett- und Schussfäden an den Kreuzungspunkten elektrisch miteinander zu verbinden oder voneinander elektrisch zu isolieren.

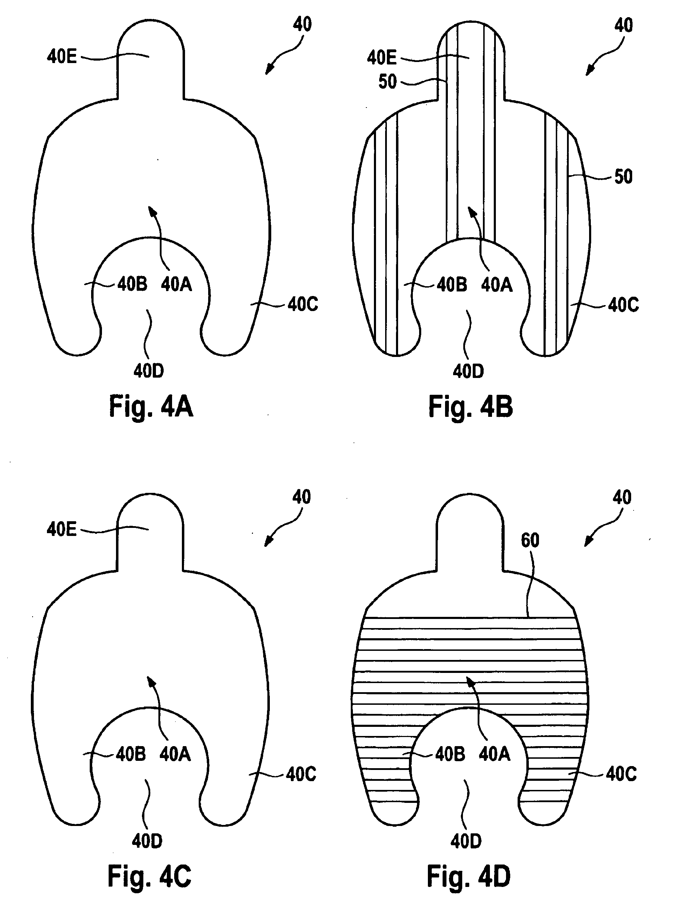

Die Fig. 4A - 4I zeigen eine weitere Ausführungsform des U-förmigen Pad 40, das aber einen zentralen Ausschnitt nicht aufweist. Der Verlauf der beiden Leiterbahnen unterscheidet sich von dem Leiterbahnverlauf des ersten Ausführungsbeispiels. Die einander entsprechenden Elemente sind mit den gleichen Bezugszeichen versehen. Bei dem zweiten Ausführungsbeispiel ist eine weitere Isolationsebene vorgesehen, die elektrisch leitfähige Kett- und Schussfäden der darüber oder darunter liegenden Ebenen voneinander trennt. Dies wird anhand der Fig. 4A - 4I deutlich.

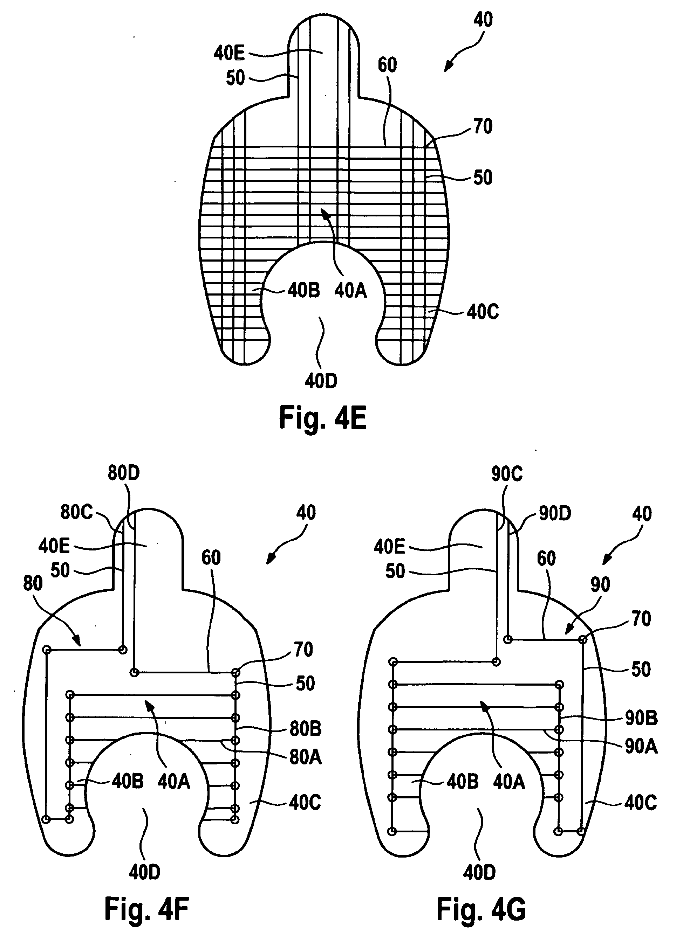

Fig. 4A zeigt die auf die Haut des Patienten aufzulegende erste Lage des Pad, die elektrisch nicht leitend ist. Auf der ersten Ebene liegt eine zweite Ebene mit elektrisch leitfähigen Kettfäden 50 (Fig. 4B). Auf der Ebene mit den elektrisch leitfähigen Kettfäden liegt eine dritte Ebene, die elektrisch nicht leitend ist, da elektrisch leitfähige Kett- und Schussfäden nicht kontaktieren (Fig. 4C). Auf der dritten Ebene, liegt eine vierte Ebene mit elektrisch leitfähigen Schussfäden 60. In der zweiten und vierten Ebene sind die elektrisch leitfähigen Kett- bzw. Schussfäden 50, 60 in unterschiedlichem Abstand zueinander angeordnet, sodass sich die in Fig. 4E dargestellte Struktur ergibt.

Die elektrisch leitfähigen Kett- und Schussfäden 50, 60, die die erste Leiterbahn 80 bilden, zeigt Fig. 4F, während Fig. 4G die elektrisch leitfähigen Kett- und Schussfäden 50, 60 zeigen, die die zweite Leiterbahn 90 bilden. Die Fig. 4F und 4G zeigen, dass der halbkreisförmige Ausschnitt 40D einen Teil der zwischen zwei elektrischen Verbindungspunkten parallel zueinander verlaufenden Abschnitte 80A, 90A der ersten und zweiten Leiterbahn 80, 90 unterbricht, die eine Parallelschaltung von elektrisch leitfähigen Fäden bilden. Bei diesem Ausführungsbeispiel weisen sowohl die erste als auch zweite Leiterbahn 80, 90 jeweils einen Leiterbahnabschnitt auf, der von mehr als zwei Fäden gebildet wird. Folglich kann die erste oder zweite Leiterbahn auch dann nicht unterbrochen werden, wenn einer der mindestens zwei Fäden dieses Leiterbahnabschnitts reißt. Die Figuren sollen verdeutlichen, dass durch die Anzahl und Ausbildung der Ausschnitte in dem Pad die Redundanz erhöht oder verringert werden kann. Zur Erhöhung der Redundanz kann die Anzahl elektrisch leitfähigen Kett- und Schussfäden, die eine Parallelschaltung bilden, bei einzelnen oder sämtlichen Leiterbahnabschnitten der Leiterbahn erhöht werden, während zur Verringerung der Redundanz die Anzahl der Kett- bzw. Schussfäden einzelner oder sämtlicher Leiterbahnabschnitte verringert werden kann.

Eine hohe Redundanz der Leiterbahnen, d.h. eine Mehrzahl von leitfähigen Fäden, führt zu einer hohen Sensitivität des Feuchtesensors, weil an jeder Stelle des Sensors bereits kleine Blutmengen zwischen den nahe beieinander liegenden Leiterbahnen erkannt werden können. Eine geringe oder keine Redundanz bedingt umgekehrt eine geringe Sensitivität. Ein Nachteil einer hohen Redundanz ist allerdings, dass bei einem Leiterbahnbruch die Fehlfunktion eines ungeprüften Sensors erst bei der Anwendung festgestellt wird, sofern nicht vorher jede einzelne Leiterbahn auf ihre Integrität überprüft wird. Bei Sensoren mit Redundanz erfolgt deshalb eine In-Prozeß-Kontrolle (IPK), bei der jede einzelne Leiterbahn im Herstellungsprozess auf ihre Funktionsfähigkeit überprüft wird.

Bei Sensoren, die keine Redundanz haben, kann ebenfalls eine In-Prozeß-Kontrolle (IPK) durchgeführt werden, bei der jede einzelne Leiterbahn im Herstellungsprozeß auf ihre Funktionsfähigkeit überprüft wird.

Wenn in dem Gewebe Fäden mit einer hohen Reißfestigkeit verwendet werden, kann eine elektrische Struktur mit einer geringeren Redundanz ausreichend sein, während bei der Verwendung von Fäden mit einer geringeren Reißfestigkeit eine elektrische Struktur mit einer hohen Redundanz vorteilhaft ist.

Im Übrigen kann die Funktionsfähigkeit des Feuchtigkeitssensors dadurch überprüft werden, dass der Widerstand zwischen den Anschlusskontakten gemessen wird. Wenn ein Leiterbahnabschnitt aus nur einem leitfähigen Faden unterbrochen wird, wird ein unendlich hoher Widerstand gemessen. Bei einer Unterbrechung eines Fadens bei einem Leiterbahnabschnitt aus mehreren Fäden, die eine Parallelschaltung bilden, kann der Defekt eines einzelnen Fadens hingegen nicht dadurch nachgewiesen werden, dass ein unendlich hoher Widerstand gemessen wird.

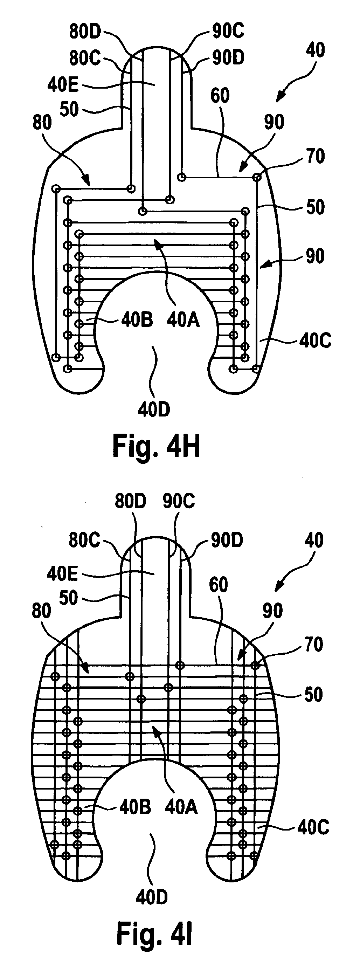

Fig. 4H zeigt zur weiteren Veranschaulichung beide Leiterbahnen 80, 90 mit den jeweiligen Anschlusskontakten 80C, 80D, 90C, 90D an der Lasche 40E des Pad 40. Fig. 4I zeigt die beide Leiterbahnen 80, 90 bildenden Kett- und Schussfäden 50, 60 über ihre gesamte Länge.

Der besseren Übersichtlichkeit halber sind in den Figuren 4F bis 4H wieder nur die Abschnitte der leitfähigen Kett- und Schussfäden eingezeichnet, die die Leiterbahnen bilden. Die Kett- und Schussfäden durchlaufen aber das Gewebe über die gesamte Breite und Länge.

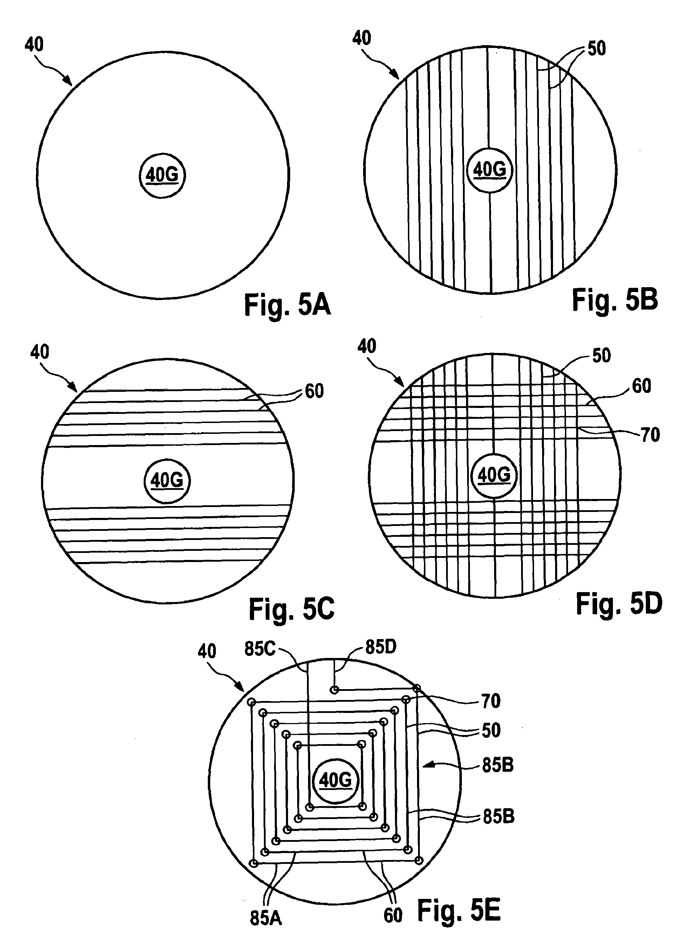

Die Fig. 5A - 5F zeigen ein weiteres Ausführungsbeispiel des Pad 40, wobei für die einander entsprechenden Elemente wieder gleiche Bezugszeichen verwendet werden. Bei dieser Ausführungsform ist das Pad 40 kreisrund und weist einen zentralen kreisförmigen Ausschnitt 40G zum Durchführen der Kanüle auf. Darüber hinaus unterscheidet sich dieses Ausführungsbeispiel von dem unter Bezugnahme auf die Fig. 3 und 4 beschriebenen Ausführungsformen dadurch, dass nur eine Leiterbahn 85 in Form einer mäanderförmigen geschlossenen Leiterschleife vorgesehen ist (Fig. 5E).

Das Pad 40 besteht aus einem dreilagigen Gewebe mit einer unteren Lage (Fig. 5A), die elektrisch nicht leitend ist, einer mittleren Lage (Fig. 5B) mit elektrisch leitfähigen Kettfäden 50 und einer oberen Lage (Fig. 5C) mit elektrisch leitfähigen Schussfäden 60. In Fig. 5D sind die sich kreuzenden Kett- und Schussfäden 50, 60 der mittleren und oberen Ebene dargestellt. Fig. 5E zeigt die als Kreise dargestellten Kontaktstellen zwischen den elektrisch leitfähigen Kett- und Schussfäden 50, 60, die sich an den Verbindungspunkten 70 kreuzen (Fig. 5D). Die Überlagerung der sich kreuzenden Kett- und Schussfäden 50, 60 ergibt eine aus einer Vielzahl von rechteckförmig zueinander verlaufenden Abschnitten 85A, 85B bestehenden Leiterschleife, bei der die Leiterbahn 85 schneckenförmig von außen nach innen verläuft. Die beiden Anschlusskontakte 85C, 85D der Leiterbahn 85 sind nach außen geführt und liegen parallel zueinander.

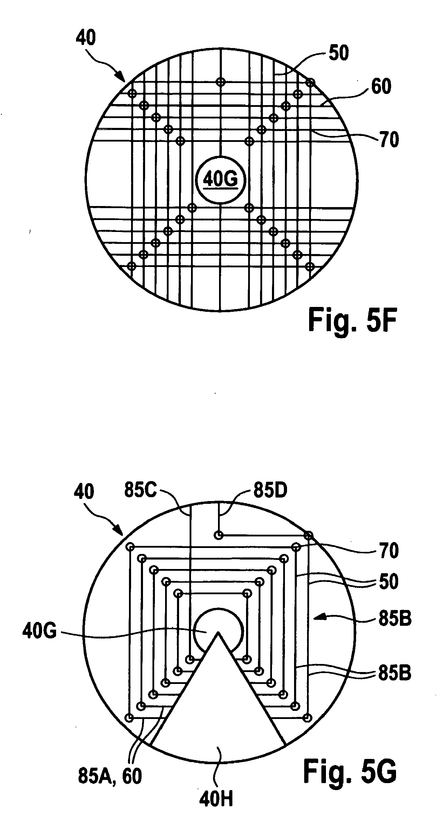

Um bei den beschriebenen Ausführungsformen des Pad 40 auf jeden Fall zu verhindern, dass die elektrisch leitfähige Punktionskanüle einen Kurzschluss zwischen einzelnen Abschnitten der Leiterbahn verursacht, kann an der Oberseite des Pad ein isolierender Gewebebereich 40H geschaffen werden, in dem keine leitfähigen Fäden an die Oberfläche treten. Fig. 5G zeigt als Beispiel einen isolierenden dreieckförmigen Gewebebereich 40H an der Oberseite des kreisförmigen Pads 40. Der Gewebebereich 40H erstreckt sich bis an den zentralen Ausschnitt 40G zum Durchführen der Nadel. Es ist aber auch jede beliebige andere Form für die Isolationsschicht möglich. Allein entscheidend ist, dass die nach außen weisende Oberfläche des Pads zumindest im Bereich unterhalb der Punktionskanüle elektrisch nicht leitend ist, sodass die metallische Punktionskanüle keinen Kurzschluss verursachen kann. Dies kann wie oben beschrieben allein durch den Webprozess erzielt werden. Eine zusätzliche lokale Isolationsschicht ist dann auf dem fertigen Gewebe nicht mehr erforderlich, wäre aber auch möglich, was jedoch Aufwand und Kosten erhöhen würde.

Fig. 5F zeigt nochmals sämtliche Kreuzungspunkte mit den elektrisch leitfähigen Kett- und Schussfäden 50, 60 über ihre gesamte Länge.

Die Vorrichtung zum Detektieren von Feuchtigkeit, die nur über eine Leiterbahn 85 mit zwei Anschlusskontakten verfügt (Fig. 5A - 5F) wird über ein zweiadriges Verbindungskabel 42 an die Auswerteinheit 41 der Überwachungsvorrichtung B angeschlossen (Fig. 2). Ein Abschlusswiderstand ist bei dieser Ausführungsform nicht erforderlich. In Abhängigkeit von der Feuchtigkeit ändert sich der Widerstand zwischen den Anschlusskontakten 85A, 85B. Wenn der Widerstand einen vorgegebenen Grenzwert überschreitet, spricht die Auswerteinheit 41 an.

Bei der Ausführungsform mit zwei Leiterbahnen 80, 90 (Fig. 3 und 4) ist hingegen ein Abschlusswiderstand R erforderlich, der das eine Ende der einen Leiterbahn mit dem anderen Ende der anderen Leiterbahn verbindet, sodass eine Leitschleife gebildet wird. Der Abschlusswiderstand R wird zwischen die innen liegenden Anschlusskontakte 90C, 90D geschaltet. An die äußeren Anschlusskontakte 80C, 80D wird ein zweiadriges Verbindungskabel 42 angeschlossen, dass den Feuchtigkeitssensor mit der Auswerteinheit 42 der Überwachungsvorrichtung B elektrisch verbindet. Der Gesamtwiderstand der Leiterschleife setzt sich dann aus der Summe der Widerstände beider Leiterbahnen 80, 90 und des Abschlusswiderstands R zusammen. Bei dem Abschlusswiderstand handelt es sich um einen sehr hochohmigen Widerstand, insbesondere um einen Widerstand von größer als 100 kOhm, während die Leiterbahnwiderstände niederohmig sind. Die elektrisch leitfähigen Fäden können als solche beispielsweise einen längenspezifischen Widerstand von 100 Ohm pro Meter Fadenlänge aufweisen. Beispielsweise sind die Leiterbahnwiderstände fertig gewebter Leiterbahnen einschließlich der Widerstände aller Verknüpfungspunkte in der Summe kleiner als 1 kOhm.

Die Auswerteinheit 42 der Überwachungsvorrichtung misst den Widerstand zwischen den Anschlusskontakten 80C, 80D. Wenn das Pad 40 mit Flüssigkeit, insbesondere Blut benetzt werden sollte, verringert sich der zwischen den Anschlusskontakten gemessene Widerstand, sodass die Auswerteinheit 41 einen Störfall detektiert.

Die Auswerteinheit 41 erlaubt auch eine Überprüfung der Funktionsfähigkeit der Detektionsvorrichtung 40. Hierzu misst die Auswerteinheit 41 den Widerstand zwischen den Anschlusskontakten. Dieser Widerstand muss der Summe von Abschlusswiderstand R und Leiterbahnwiderstand entsprechen, wenn das Pad 40 nicht mit Flüssigkeit benetzt ist. Wenn der gemessene Widerstand um ein vorgegebene Differenz von dem Abschlusswiderstand abweichen sollte, stellt die Auswerteinheit fest, dass die Detektionsvorrichtung 40 nicht funktionsfähig, d.h. eine Leiterbahn unterbrochen ist.

Die erfindungsgemäße Detektionsvorrichtung 40 mit den beiden Leiterbahnen hat den Vorteil, dass die besondere Leiterbahnführung die Verlagerung des Abschlusswiderstands R außerhalb des Pads erlaubt. Dadurch ist eine einfachere Fertigung des Pad möglich. Denn in einem Webprozess ließe sich der Abschlusswiderstand nicht mit ausreichender Reproduzierbarkeit herstellen. Das Pad kann also ohne zusätzliche Verfahrensschritte allein durch Weben hergestellt werden. Ein Abschlusswiderstand braucht auch nach dem Webprozess nicht auf das Pad aufgebracht zu werden. Dadurch ergibt sich der Vorteil eines stets reprodzierbaren Abschlusswiderstands unabhängig von dem Webprozess.

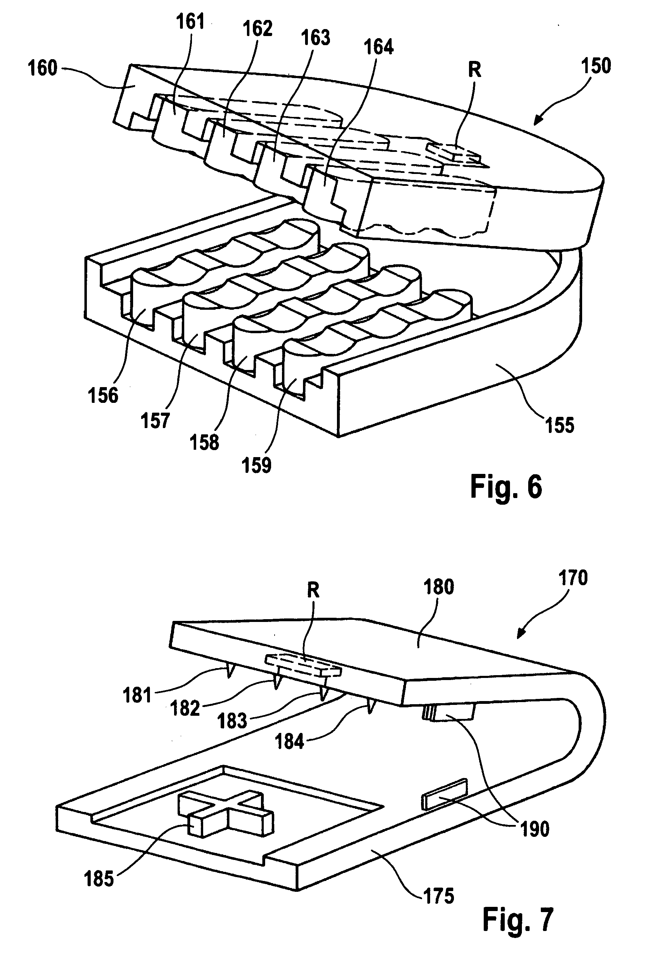

Fig.6 zeigt eine Prinzipdarstellung der wesentlichen Elemente eines Anschlussteils 150 zum Anschluss des Pads 40 von den Figuren 4 ohne kreuzförmigen Ausschnitt an die Auswerteinheit 41 der Überwachungsvorrichtung B. Grundsätzlich kann aber auch das Pad 40 von den Figuren 3 mit kreuzförmiger Ausnehmung an den Anschlussteil 150 angeschlossen werden. Dann kann aber die kreuzförmige Ausnehmung nicht zur Fixierung des Pads dienen.

Der Anschlussteil 150 ist als Klemmvorrichtung zum Verklemmen der Lasche 40E des Pads 40 ausgebildet. Er weist einen unteren Klemmteil 155 und einen oberen Klemmteil 160 auf, wobei in dem unteren Klemmteil 155 vier nebeneinander liegende Anschlusskontakte 156, 157, 158, 159 und in dem oberen Klemmteil 160 vier nebeneinander liegende Anschlusskontakte 161, 162, 163, 164 angeordnet sind. Der obere und untere Klemmteil 155, 160 können miteinander verklemmt werden, wobei die Lasche 40E des Pads 40 mit den Anschlusskontakten 80C, 80D, 90C, 90D zwischen den einander gegenüberliegenden Anschlusskontakten 156, 157, 158, 159 und 161, 162, 163, 164 des oberen und unteren Klemmteils 155, 160 liegen. Die beiden innen liegenden Anschlusskontakte 162, 163 des oberen Klemmteils 160 sind über einen nur schematisch dargestellten Abschlusswiderstand R elektrisch miteinander verbunden. Der Abschlusswiderstand R kann ein in den oberen Klemmteil 160 integrierter SMD-Widerstand (Miniaturwiderstand) sein.

Fig.7 zeigt eine Prinzipdarstellung eines zweiten Ausführungsbeispiels des als Klemmvorrichtung ausgebildeten Anschlussteils 170. Der Anschlussteil 170 weist elastisch miteinander verbundene Schenkel 175, 180 auf, wobei der eine Schenkel 175 länger als der andere Schenkel 180 ist.

Der in Fig.7 dargestellte längere untere Schenkel 175 des Anschlussteils 170 weist einen vorspringenden Ansatz 185 auf, der in der Form einem Ausschnitt eines Pads entspricht. Bei dem vorliegenden Ausführungsbeispiel ist der vorspringende Ansatz 185 kreuzförmig, da das nicht dargestellte passende Pad einen zentralen kreuzförmigen Ausschnitt 40F aufweist. Es ist aber auch jede beliebige andere Form möglich.

Der kürzere obere Schenkel 180 weist an der Unterseite vier nebeneinander liegende Anschlusskontakte 181, 182, 183, 184 auf, die als Dorne ausgebildet sind. An den gegenüberliegenden Innenseiten der beiden Schenkel 175, 180 sind nur andeutungsweise dargestellte Rastmittel 190 vorgesehen, sodass die Schenkel nach dem Zusammendrücken einrastend festgelegt sind. Auch bei dieser Ausführungsform sind die beiden innen liegenden Anschlusskontakte 182, 183 des Anschlussteils 170 über einen Abschlusswiderstand R verbunden, der als in den oberen Schenkel 180 integrierter SMD-Widerstand ausgebildet ist.

Zum Anschluss der Detektionsvorrichtung 40 an die Überwachungsvorrichtung B wird das nicht dargestellte Pad zwischen die beiden Schenkel 175, 180 des Anschlussteils 170 gelegt, sodass der kreuzförmige Ansatz 185 in den kreuzförmigen Ausschnitt 40F des Pads 40 greift. Anschließend werden die beiden Schenkel 175, 180 des Anschlussteils 180 zusammengedrückt, wobei die Anschlusskontakte 181, 182, 183, 184 des Anschlussteils 170 mit den Anschlusskontakten 80C, 80D, 90C, 90D des Pads kontaktieren. Dabei wird das Pad von den domförmigen Anschlusskontakten 181, 182, 183, 184 fixiert.

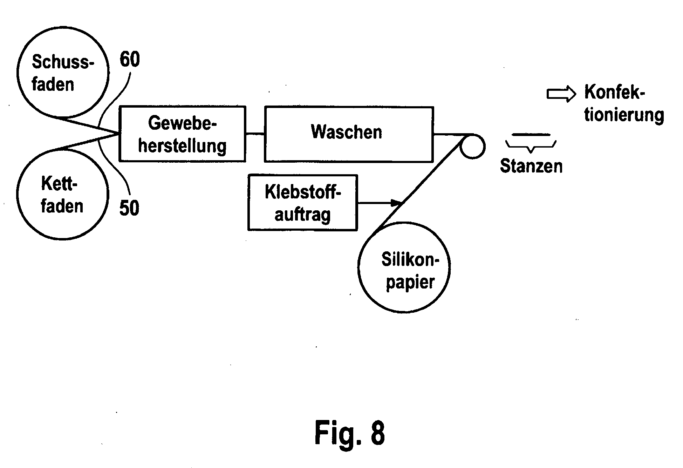

Fig.8 zeigt die wesentlichen Verfahrensschritte des Webprozesses zur Herstellung der erfindungsgemäßen Detektionsvorrichtung. Zur Herstellung des vorzugsweise mehrlagigen Gewebes werden die Kettfäden 50 und Schussfäden 60 zugeführt. Nach der Herstellung des Gewebes erfolgen weitere dem Fachmann bekannte Verfahrensschritte, zu denen das Waschen zählt. Anschließend wird auf die Unterseite der Gewebebahn ein Deckmaterial mit einer Adhäsions- oder Klebstoffschicht aufgetragen. Der Klebstoff kann beispielsweise mit einer Walzenbürste aufgetragen werden. Vorzugsweise wird ein mit Klebstoff beschichtetes Silikonpapier auf die Rückseite der Gewebebahn aufgetragen. Alternativ und besonders bevorzugt wird eine beidseitig selbstklebende Klebefolie, beispielsweise eine PET-Folie, auf die Rückseite der Gewebebahn aufgetragen. Die Funktion einer Klebefolie liegt einerseits in der Bereitstellung einer Barriere gegen Durchnässung des Pflastersensors mit Schweiß des Patienten. Andererseits kann mit einer beidseitig klebenden Klebefolie eine unterschiedliche Klebkraft an der Ober- und Unterseite vorgesehen werden. Vorzugsweise weist die Folie an der der Haut des Patienten zugewandten Seite eine kleinere Klebkraft als der der Haut abgewandten und dem Gewebe zugewandten Seite auf. Auf der der Haut zugewandten Seite weist die Klebefolie vorzugsweise ein Silikonpapier zum Schutz der Klebstoffschicht auf.

Anstelle von Silikonpapier kann auch eine silikonisierte Kunststoff-Folie eingesetzt werden. Entscheidend ist, dass die Klebeschicht des Sensors von dem Silikonpapier oder der silikonisierten Kunststoff-Folie leicht ablösbar ist.

Daraufhin werden die Pads vereinzelt, beispielsweise durch Stanzen oder Schneiden von der Gewebebahn abgetrennt. In dem Stanz- oder Schneidprozess können auch die Ausschnitte des Pads hergestellt werden.

Die Pads können einzeln steril verpackt oder mehrere Pads können übereinanderliegend steril verpackt werden. Eine sterile Verpackung der Pads ist aber nicht zwingend erforderlich. Beim Einsatz des Pads zur Überwachung eines Zentralvenenkathethers wird vorzugsweise ein steriles Pad eingesetzt, das beispielsweise mit den bekannten Sterilisationsverfahren ETO (Ethylenoxid) oder E-Beam (Electron Beam Sterilisation) sterilsiert wurde. Alternativ kann auch eine Dampfsterilisierung vorgenommen werden.

Zum Gebrauch wird das Deckmaterial von dem Pad abgezogen und das Pad mit der Adhäsions- oder Klebeschicht auf die Haut des Patienten aufgelegt. Anschließend kann die Punktion mit der Kanüle erfolgen. Es ist aber auch möglich, das Pad nach der Punktion auf die Haut des Patienten aufzulegen, wenn das Pad seitlich ausgeschnitten ist. Es ist möglich den Anschlussteil vor oder nach dem Auflegen des Pads auf die Haut an das Pad anzuschließen.

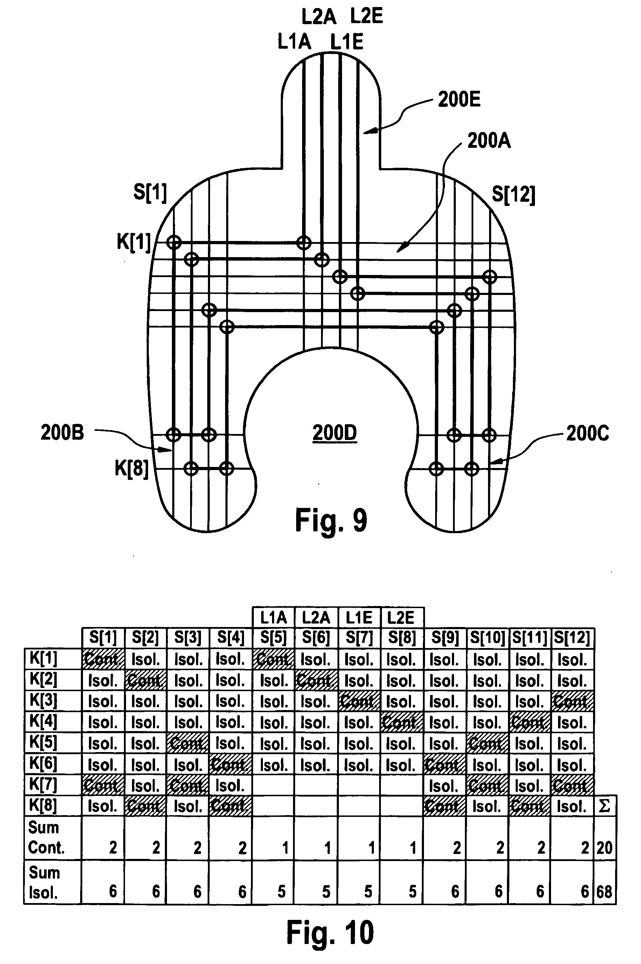

Fig. 9 zeigt ein weiteres Ausführungsbeispiel der Vorrichtung zum Detektieren von Feuchtigkeit in schematischer Darstellung, die nachfolgend wieder als Pad bezeichnet wird. Das Pad hat mit Ausnahme des zentralen Ausschnitts die gleiche Form wie das unter Bezugnahme auf die Figuren 3A bis 3H beschriebene Pad. Es weist einen mittleren Bereich 200A mit zwei Schenkeln 200B, 200C auf, die einen halbkreisförmigen Ausschnitt 200D seitlich umschließen. An den mittleren Bereich ist eine Lasche 200E angeformt, die beiden Schenkeln gegenüberliegt.

Die eine elektrisch leitende Struktur bildenden elektrisch leitfähigen Kett- und Schussfäden sind durch horizontale und vertikale dünne Linien gekennzeichnet. Bei dieser Ausführungsform verlaufen im Gegensatz zu den oben beschriebenen Ausführungsbeispielen die Schussfäden S in vertikaler und die Kettfäden K in horizontaler Richtung. Die elektrisch leitende Struktur wird von 8 Kettfäden K [1] bis K [8] und 12 Schussfäden S[1] bis S [12] gebildet, die an den Kreuzungspunkten derart angeordnet sind, dass sie entweder elektrisch leitend verbunden sind oder elektrisch voneinander isoliert sind.

Fig. 10 zeigt eine Matrix zur Veranschaulichung der 88 Kreuzungspunkte der 8 Kettfäden K [1] bis K [8] und 12 Schussfäden S [1] bis S [12]. Die Schnittpunkte zweier leitfähiger Fäden, die einen Kontakt erzeugen, sind in der Matrix mit "Cont." bezeichnet, während die Schnittpunkte zweier leitfähiger Fäden, die eine Isolationsstelle bilden, mit "Isol." bezeichnet sind. Es ergibt sich eine elektrisch leitende Struktur, die zwei Leiterbahnen aufweist, die jeweils eine nicht redundant ausgeführte Leiterschleife bilden.

In Fig. 9 sind die elektrischen Kontaktstellen an den Kreuzungspunkten zwischen den elektrisch leitfähigen Kett- und Schussfäden K [i], S [i] als Kreise dargestellt. Die erste Leiterbahn L1A-L1E verläuft von der Lasche 200D über den mittleren Bereich 200A zu dem linken Schenkel 200B und von dem linken Schenkel über den mittleren Bereich zu dem rechten Schenkel 200C und von dem rechten Schenkel über den mittleren Bereich zurück zu der Lasche des Pads. Der Anfang der jeweiligen Leiterbahn ist mit "A" und das Ende der Leiterbahn ist mit "E" bezeichnet. Die beiden Enden L1A, L1E der ersten Leiterbahn L1A-L1E bilden die beiden Anschlusskontakte. Die zweite Leiterbahn L2A-L2E verläuft von der Lasche 200D über den mittleren Bereich 200A zu dem linken Schenkel 200B und von dem linken Schenkel über den mittleren Bereich zu dem rechten Schenkel 200C und von dem rechten Schenkel über den mittleren Bereich zu der Lasche des Pad 40. Die beiden Enden L2A, L2E der zweiten Leiterbahn L2A-L2E bilden das zweite Paar von Anschlusskontakten. An der Lasche 200D sind die Anschlusskontakte so angeordnet, dass die Anschlusskontakte L2A und L1E zwischen den Anschlusskontakten L1A und L2E liegen.

Das Gewebe der Ausführungsform von Fig. 9 kann ein sich über den gesamten Sensor erstreckendes dreilagiges Gewebe sein, das eine erste nicht leitfähige Lage, eine zweite leitfähige Lage mit in einer ersten Richtung leitfähigen Fäden und eine dritte leitfähige Lage mit in einer zweiten Richtung leitfähigen Fäden aufweist, wobei die zweite Richtung im Wesentlichen senkrecht zur ersten Richtung steht.

Eine alternative Ausführungsform sieht ein Gewebe vor, bei dem die Anzahl der Lagen lokal unterschiedlich ist. So kann das Gewebe in einzelnen Bereichen des Sensors eine unterschiedliche Anzahl von Lagen aufweisen. Dabei können drei unterschiedliche Bereiche ausgebildet werden, wobei der erste Bereich eine Kontaktstelle bildet, an der sich leitfähige Fäden kontaktierend kreuzen, der zweite Bereich eine Isolationsstelle bildet, bei der sich zwischen leitfähigen Fäden ein isolierender Faden befindet, und der dritte Bereich weder eine Kontaktstelle noch eine Isolationsstelle bildet.

Eine besonders bevorzugte Ausführungsform sieht vor, dass lokale Bereiche, die eine Kontaktstelle bilden, und lokale Bereiche, die weder eine Kontakt- noch eine Isolationsstelle bilden, aus insgesamt zwei Lagen bestehen. In der ersten Lage befinden sich sowohl die in der ersten Richtung als auch in der zweiten Richtung verlaufenden leitfähigen Fäden. Die zweite (oberste) Lage bildet eine nicht leitfähige Deckschicht, die dafür sorgt, das der Sensor für Berührungen vorteilhafterweise nicht empfindlich ist. Wenn der Sensor berührungsempfindlich wäre, hätte das Berühren der freiliegenden Sensorfläche des am Patienten aufgeklebten Sensors, beispielsweise mit den Fingern, einen Fehlalarm zur Folge. Eine solche Berührung des Sensors kann beispielsweise durch den Patienten selbst oder das medizinische Personal erfolgen. Die Fäden dieser isolierenden zweiten Lage tauchen partiell in die erste Lage ein, wodurch ein mechanischer Verbund zwischen der ersten und zweiten Lage hergestellt wird.

Die besonders bevorzugte Ausführungsform sieht ebenfalls einen eine Isolationsstelle bildenden lokalen Bereich vor, die insgesamt vier Lagen aufweist. In der ersten (untersten) Lage befinden sich in der ersten Richtung verlaufenden leitfähigen Fäden. Die zweite Lage weist eine Schicht aus nichtleitfähigen Fäden auf, welche die erste von der dritten Lage isoliert. In der dritten Lage befinden sich die in der zweiten Richtung verlaufenden leitfähigen Fäden. Die vierte (oberste) Lage wird von einer nicht leitfähigen Deckschicht gebildet, die den Sensor vorteilhafterweise gegen Berührung unempfindlich macht.

Die oben beschriebene Isolationsstelle kann beispielsweise an den Stellen vorgesehen sein, die in Fig. 10 mit "Isol" bezeichnet sind. Bei diesem Ausführungsbeispiel ergeben sich 66 Isolationsstellen. Die Isolation zur Haut wird bei dieser Ausführungsform mit einer isolierenden Klebefolie erreicht.

Das Pad mit den lokal unterschiedlichen Bereichen weist eine unterschiedliche Dicke auf. Ein besonderer Vorteil dieser Ausführungsform liegt in der Materialeinsparung, weil das Pad nur an den Stellen eine ausreichende Dicke aufweisen muss, wo elektrisch leitfähige Fäden voneinander isoliert werden müssen. Die Materialeinsparung erlaubt eine besonders kostengünstige Herstellung des Sensors.

Das Pad von Fig. 9 kann an einen Anschlussteil angeschlossen werden, der sich von dem unter Bezugnahme von Fig. 6 beschriebenen Anschlussteil nur dadurch unterscheidet, dass der Abschlusswiderstand R mit einem innen liegenden und einem außen liegenden Anschlusskontakt verbunden ist.

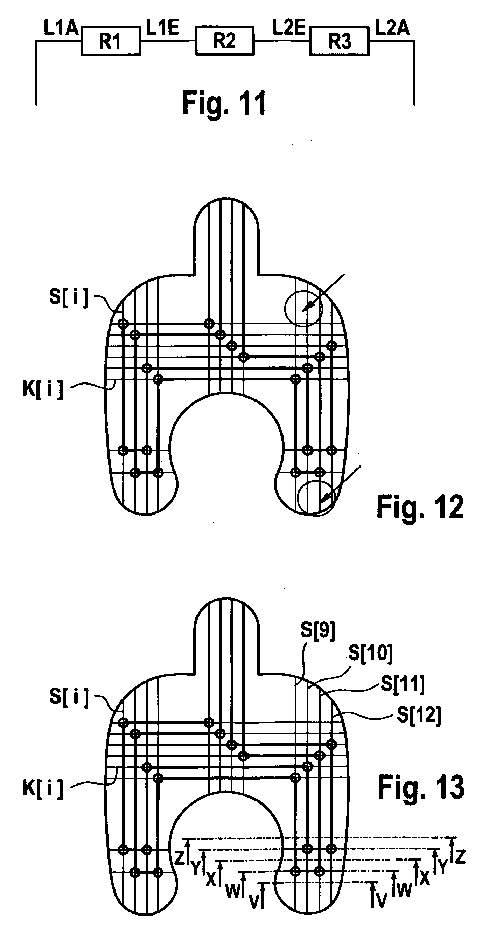

Fig. 11 zeigt das elektrische Ersatzschaltbild der an den Anschlussteil angeschlossenen elektrisch leitenden Struktur des Pads. Das Ersatzschaltbild ist eine Serienschaltung des Widerstands R1 der ersten Leiterbahn L1A-L1E, des Widerstands R2 des Abschlusswiderstands und des Widerstands R3 der zweiten Leiterbahn L2A-L2E. Die Widerstande R1 und R3 der ersten und zweiten Leiterbahn sollten vorzugsweise jeweils nicht größer als 200 Ω sein.

Die Sensitivität des Pad für Feuchtigkeit ist nicht nur unmittelbar in dem Bereich der leitenden Struktur der Kett- und Schussfäden, sondern auch in den Randbereichen des Pads gegeben, da sich die Kett- und Schussfäden bis an den Rand des Pads erstrecken. In Fig. 12 sind als Beispiel zwei Randbereiche des Pad mit Kreisen gekennzeichnet, in denen das Pad für Feuchtigkeit sensitiv ist. Im Übrigen kann die Empfindlichkeit des Pads für Feuchtigkeit mit einer nicht leitfähigen gewebten Deckschicht eingestellt werden.

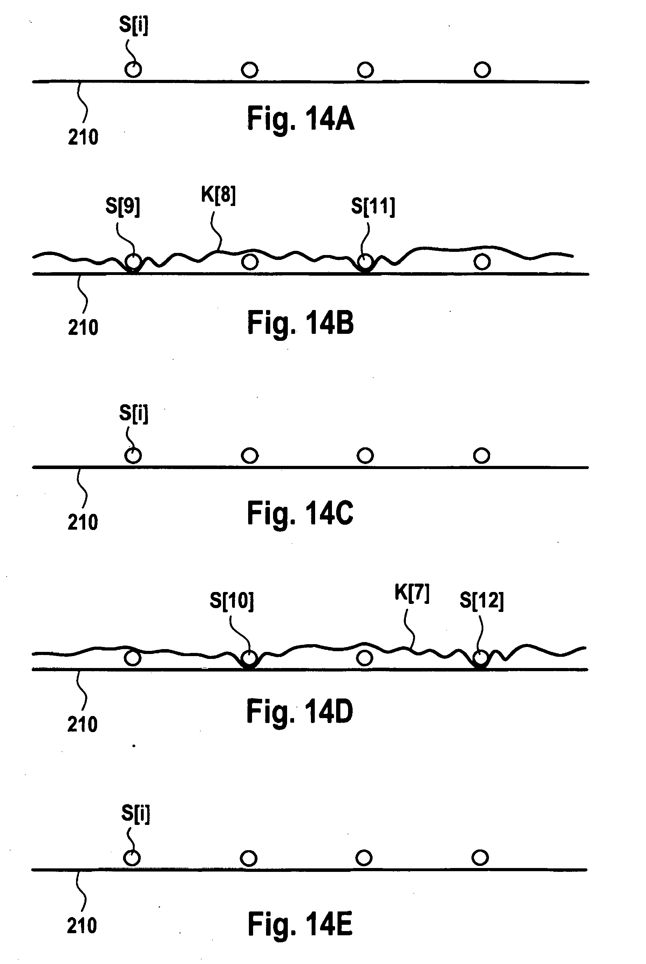

Die Fig. 14A bis 14E zeigen einen Schnitt durch die Abdeckung 210 (Liner), beispielsweise ein Silikonpapier, und die Verknüpfungen der Kett- und Schussfäden des Pads in den Schnittebenen, die in Fig. 13 dargestellt sind. In der Schnittebene V - V sind die Kettfäden K [i] und Schussfäden S [i] nicht verknüpft, da Kettfäden in dieser Ebene nicht vorhanden sind. In der Schnittebene W - W ist der Kettfaden K [8] mit dem Schussfaden S [9] sowie der Kettfaden K[8] mit dem Schussfaden S [11] verknüpft, so dass eine elektrische Verbindung zwischen Kett- und Schussfaden hergestellt wird. In der Schnittebene X - X sind die Kettfäden K [i] und Schussfäden S [i] nicht verknüpft, da Kettfäden in dieser Ebene nicht vorhanden sind. In der Schnittebene Y - Y ist der Kettfaden K [7] mit dem Schussfaden S [10] sowie der Kettfaden K [7] mit dem Schussfaden S [12] verknüpft, so dass eine elektrische Verbindung zwischen Kett- und Schussfaden hergestellt wird. In der Schnittebene Z - Z sind die Kettfäden K [i] und Schussfäden S [i] nicht verknüpft, da Kettfäden in dieser Ebene nicht vorhanden sind.

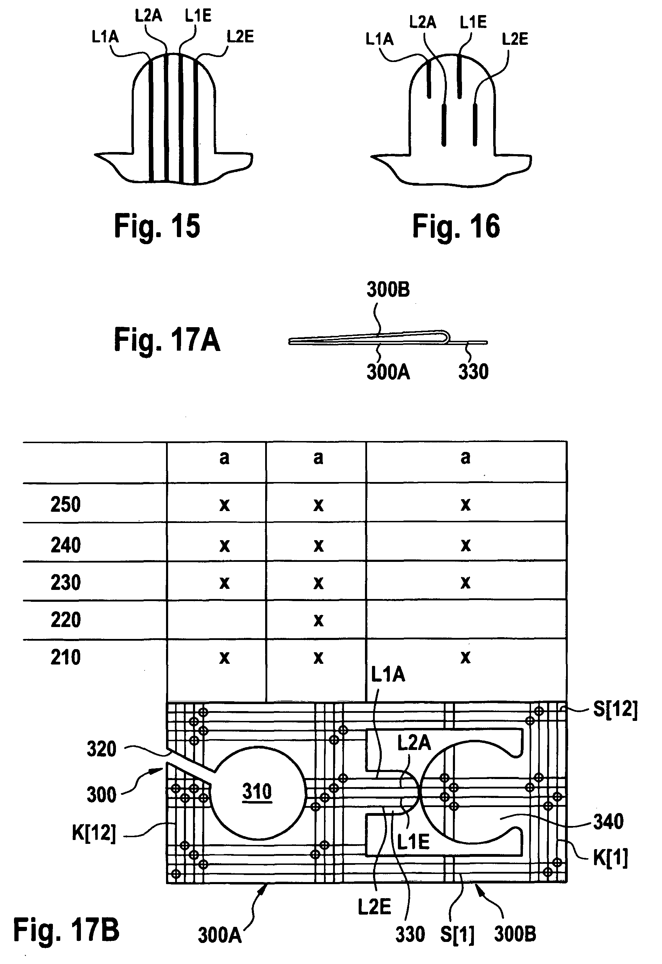

Fig. 15 zeigt in schematischer Darstellung die Anordnung der Anschlusskontakte L1A, L1E und L2A, L2E an der Lasche 200D der oben beschriebenen Ausführungsformen des Pads. Die Enden der Kett- oder Schussfäden verlaufen bei diesen Ausführungsformen in gleichem Abstand bis an den Rand der Lasche. Zur Bildung der Anschlusskontakte L1A, L1E und L2A, L2E befinden sich die Fäden an der Oberfläche der Lasche 200D. Um einen Kurzschluss zwischen den Anschlusskontakten des Anschlussteils zu vermeiden, muss die Breite oder der Durchmesser der Anschusskontakte des Anschlussteils kleiner als der Abstand zwischen den Anschlusskontakten L1A und L2A, L2A und L1E sowie L1E und L2E des Pads sein. Damit ist die Breite bzw. der Durchmesser der Anschlusskontakte des Anschlussteils beschränkt.

Fig. 16 zeigt eine alternative Ausführungsform der Anordnung der Anschlusskontakte an der Lasche 200D des Pads in schematischer Darstellung, bei der die Anschlusskontakte L1A und L1E der einen Leiterbahn L1A-L1E zu den Anschlusskontakten L2A und L2E der anderen Leiterbahn L2A-L2E versetzt angeordnet sind. Die Anschlusskontakte L1A und L1E der einen Leiterbahn befinden sich dabei auf der oberen Hälfte und die Anschlusskontakte L2A und L2E der anderen Leiterbahn auf der unteren Hälfte der Lasche 200D. Da das Pad an der Oberfläche eine isolierende gewebte Deckschicht aufweist, ist ein gezieltes "Abtauchen" der Fäden möglich. Bei der Ausführungsform von Fig. 16 liegen die Schussfäden S [5] und S [7] (Fig. 9) in der unteren Hälfte der Lasche unterhalb der Deckschicht und die Schussfäden S [6] und S [8] (Fig. 9) in der oberen Hälfte der Lasche unterhalb der Deckschicht, so dass die Anschlusskontakte des Anschlussteils eine größere Breite oder einen größeren Durchmesser als bei dem Ausführungsbeispiel von Fig. 15 haben können, ohne dass ein Kurzschluss zwischen den Kontakten L1A und L1E bzw. L2A und L2E entsteht.

Nachfolgend werden weitere alternative Ausführungsformen des Pads beschrieben, die sich in der Form und dem Verlauf der Leiterbahnen voneinander unterscheiden. Fig. 17A zeigt ein sich in zwei Hälften aufteilendes Pad 300 in einer Seitenansicht, bei dem die eine Hälfte nach dem Auflegen auf die Haut des Patienten auf die andere Hälfte umgeklappt wird. Fig. 17A zeigt die Seitenansicht des Pads nach dem Umklappen.

In Fig. 17B ist das Pad von Fig. 17A vor dem Umklappen in der Draufsicht dargestellt. Die erste Hälfte des Pad, die auf der Haut des Patienten zu liegen kommt, ist mit 300A bezeichnet, während die umzuklappende zweite Hälfte des Pad mit 300B bezeichnet ist. Das Pad besteht aus mehreren Lagen, die in Fig. 17B in Form einer dem Pad zugeordneten Tabelle beschrieben werden, die den einzelnen Bereichen des Pad zugeordnete Zeilen bzw. Spalten aufweist. Die erste Hälfte 300A des Pad 300 ist in zwei Felder (zwei Spalten in der Tabelle) gleicher Größe unterteilt. Der zweiten Hälfte 300B des Pad 300 ist ein Feld (eine Spalte in der Tabelle) zugeordnet. Die Zeilen in der Tabelle kennzeichnen die einzelnen Lagen.

Die unterste Lage bildet eine Abdeckung 210, beispielsweise eine Abziehfolie, mit der eine auf der Haut des Patienten haftende Adhäsionsschicht 220 abgedeckt wird. Wie aus der Tabelle ersichtlich ist, befindet sich die Abdeckung 210 an der Unterseite der beiden Hälften 300A und 300B des Pad 300, da alle Felder mit "X" gekennzeichnet sind. Die Adhäsionsschicht 220 befindet sich hingegen nur in dem mittleren Bereich der ersten Hälfte 300A des Pad 300.

Nach der Adhäsionschicht 220 folgt eine für Feuchtigkeit bzw. Flüssigkeit undurchlässige Lage 230, beispielsweise eine PET-Folie, die sich über die gesamte Fläche des Pad erstreckt. Auf der Oberseite der PET-Folie befindet sich eine adhäsive Beschichtung 240, auf der ein mehrlagiges Gewebe 250 mit einer elektrisch leitenden Struktur liegt, die von 12 Kettfäden K [1] bis K [12] und 12 Schussfäden S[1] bis S [12] gebildet wird.

Die Schnittpunkte, an denen eine elektrische Verbindung zwischen den Kett- und Schussfäden hergestellt wird, sind in Fig. 17B wieder mit einem Kreis gekennzeichnet. Die sich kreuzenden Kett- und Schussfäden sind wieder derart angeordnet, dass sie eine erste und eine zweite Leiterbahn bilden, deren Enden die Anschlusskontakte L1A, L1E und L2A, L2E des Pad 300 bilden. Die Leiterbahnen sind dabei derart angeordnet, dass die beiden Stromkreise nicht redundant sind.

Die erste Hälfte 300A des rechteckförmigen Pads 300 weist einen zentralen kreisförmigen Ausschnitt 310 auf, von dem sich ein schräg verlaufender schmaler Ausschnitt 320 bis zu der Schmalseite der ersten Hälfte des Pads erstreckt. Die zweite Hälfte 300B des Pads 300 ist derart ausgeschnitten, dass sich auf der Innenseite eine Lasche 330 für die Anschlusskontakte L1A, L1E und L2A, L2E und auf der Außenseite eine kreisförmige Abdeckung 340 für den kreisförmigen Ausschnitt 310 der ersten Hälfte des Pads ergibt. Die kreisförmige Abdeckung 340 der zweiten Hälfte ist dabei größer als der kreisförmige Ausschnitt 310 der ersten Hälfte, so dass der kreisförmige Ausschnitt der ersten Hälfte von der kreisförmigen Abdeckung vollständig abgedeckt wird, wenn die zweite Hälfte des Pad auf die erste Hälfte geklappt wird.

Das Pad 300 wird wie folgt verwendet. Nach dem Legen der nicht dargestellten Kanüle und dem Abziehen der Abziehfolie 210 wird das Pad mit der Adhäsionsschicht 220 auf die Haut des Patienten aufgeklebt. Da das Pad seitlich eingeschnitten ist, kann das Pad seitlich über die bereits gelegte Kanüle geschoben werden, so dass die Kanüle in dem kreisförmigen Ausschnitt 310 der ersten Hälfte des Pad liegt. Nunmehr wird die zweite Hälfte 300B des Pads auf die erste Hälfte 300A geklappt (Fig. 17A). Da nur der mittlere Bereich der ersten Hälfte des Pads an der Haut des Patienten haftet, kann die zweite Hälfte des Pads hierzu leicht erfasst werden. Die zweite Hälfte 300B kann an der Punktionsstelle beispielsweise mit einem Klebeband auf der Haut fixiert werden. Nach dem Falten des Pads liegt die Lasche 330 mit den Anschlusskontakten L1A, L1E und L2A, L2E frei, so dass der Anschlussteil angeschlossen werden kann.

Bei dem Pad befindet sich die elektrisch leitende Struktur mit den Kett- und Schussfäden an der Oberseite des Pad, so dass das Pad an der Oberseite sensitiv ist. Dies wird aus der Tabelle durch die Bezeichnung "a" deutlich, die für Sensitivität an der Oberseite steht. Nach dem Umklappen der zweiten Hälfte 300B auf die erste Hälfte 300B des Pads 300 wird das Pad in dem Bereich des kreisförmigen Ausschnitts 310, der sich in unmittelbarer Nähe zur Punktionsstelle befindet, auch an der Unterseite sensitiv, da dieser Bereich von der vor dem Falten an der Oberseite sensitiven Abdeckung 340 abgedeckt wird.

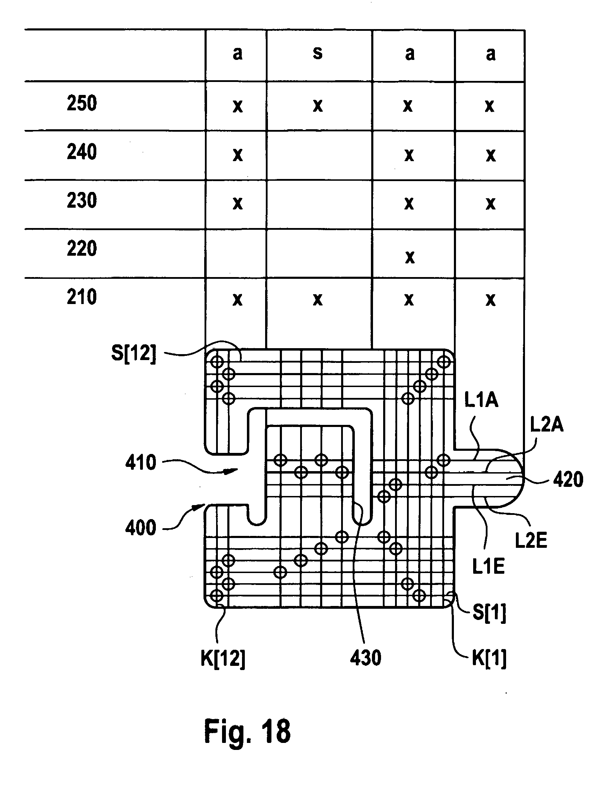

Fig. 18 zeigt eine weitere Ausführungsform des Pads 400, das aber im Gegensatz zu dem Pad 300 von den Fig. 17A und Fig. 17B nicht umgeklappt wird. Die einander entsprechenden Teile sind mit den gleichen Bezugszeichen versehen.

Der Aufbau des mehrlagigen Pads ergibt sich aus der Tabelle und der Darstellung der Schnittpunkte, an denen eine elektrische Verbindung zwischen Kett- und Schussfäden hergestellt wird. Die sich kreuzenden Kett- und Schussfäden sind wieder derart angeordnet, dass sie eine erste und eine zweite Leiterbahn bilden, deren Enden die Anschlusskontakte des Pads sind. Die elektrisch leitende Struktur wird von 12 Kettfäden K [1] bis K [12] und 12 Schussfäden S[1] bis S [12] gebildet.

Das Pad 400 ist bei dem Ausführungsbeispiel von Fig. 18 im Wesentlichen rechteckförmig. An einer Seite weist das Pad einen, beispielsweise rechteckförmigen Ausschnitt 410 auf, während das Pad an der dem Ausschnitt gegenüberliegenden Seite eine Lasche 420 aufweist. Der rechteckförmige Ausschnitt 410 an der einen Seite des Pads setzt sich in einen schmalen Spalt 430 fort, der die Leiterbahnen durchtrennt, so dass die beiden Stromkreise der elektrisch leitenden Struktur nicht redundant sind. Die Breite des Spalts 430 ist so bemessen, dass Stoßstellen von leitfähigen Fäden keinen Kurzschluss verursachen können. Bei dem Ausführungsbeispiel hat der Spalt 430 beispielsweise einen U-förmigen Verlauf, wobei der Spalt einen mittleren Bereich 440 des Pads teilweise umschließt, der auf die Punktionsstelle zu liegen kommt.

Der Tabelle ist zu entnehmen, dass das Pad 400 im mittleren Bereich 440, der von dem schmalen Spalt 430 umschlossen wird, an der Unterseite sensitiv ist, da dieser Bereich in der Tabelle mit "s" bezeichnet ist, was für eine Sensitivität an der Unterseite steht. In den übrigen Bereichen ist das Pad hingegen an der Oberseite sensitiv ("a"). Die Sensitivität an der Unterseite des Pads wird dadurch erreicht, dass die für Wasser und Feuchtigkeit undurchlässige PET-Folie 230 im mittleren Bereich 440 nicht vorhanden ist, was sich aus der Tabelle ergibt. In diesem Bereich fehlen auch die Adhäsionschicht 220 und die adhäsive Beschichtung 240 (Tabelle).

Der Vorteil dieser Ausführungsform liegt darin, dass die Punktionsstelle zusätzlich mit einem an der Unterseite sensitiven Gewebe bedeckt wird, so dass das Pad beidseitig sensitiv ist. Leckblutungen an der Punktionsstelle können mit dem auch an der Unterseite senitiven Pad sofort und zuverlässig erkannt werden können. Da die übrigen Bereiche an der Oberseite sensitiv sind, kann die unter dem Pad liegende Kanüle keinen Kurzschluss verursachen.

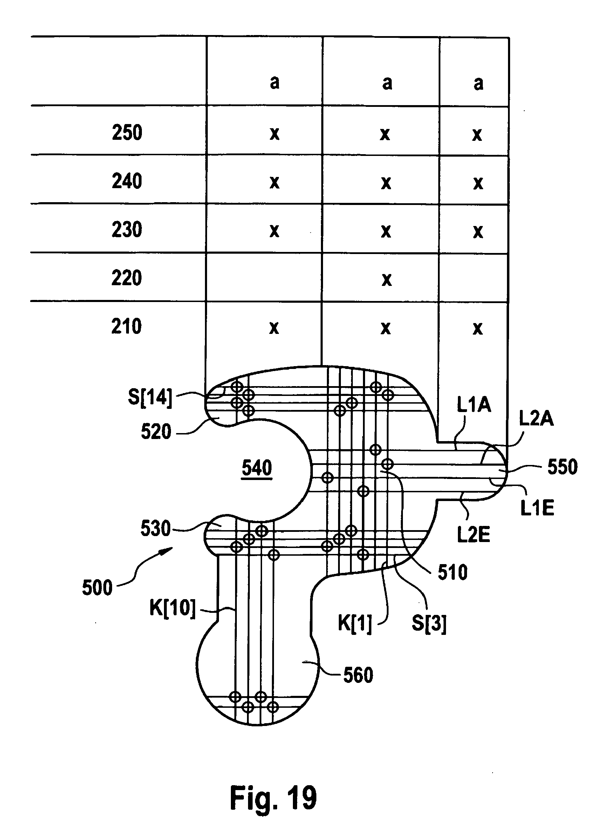

Eine weitere Ausführungsform eines beidseitig sensitiven Pads 500, das aber umgeklappt wird, zeigt Fig. 19. Die einander entsprechenden Teile sind wieder mit den gleichen Bezugszeichen versehen. Die sich kreuzenden Kett- und Schussfäden wieder derart angeordnet sind, dass sie eine erste und eine zweite Leiterbahn bilden, deren Enden die Anschlusskontakte des Pad bilden, wobei die beiden Stromkreise nicht redundant sind. Die elektrisch leitende Struktur wird von 10 Kettfäden K [1] bis K [10] und 14 Schussfäden S[1] bis S[14] gebildet

Das Pad weist einen mittleren Abschnitt 510 mit zwei Schenkeln 520, 530 auf, die einen halbkreisförmigen Ausschnitt 540 seitlich umschließen. An den mittleren Abschnitt 510 ist eine den beiden Schenkeln 520, 530 gegenüber liegende Lasche 550 mit den Anschlusskontakten L1A, L1E und L2A, L2E angeformt. Das Pad 500 weist weiterhin eine seitliche Abdeckung 560 für den halbkreisförmigen Ausschnitt 540 auf, die an einen der beiden Schenkel 520, 530 angeformt ist. Die seitliche Abdeckung 560 ist so bemessen, dass der halbkreisförmige Ausschnitt 540 des Pads, in dem die Kanüle zu liegen kommt, nach dem Umklappen der Abdeckung vollständig abgedeckt wird.

Wie sich aus der Tabelle ergibt, ist das Pad 500 vor dem Umklappen der Abdeckung 560 nur an der Oberseite sensitiv. Nach dem Umklappen der Abdeckung ist das Pad im Bereich des halbkreisförmigen Ausschnitts 540 auch an der Unterseite sensitiv, so dass an der Punktionsstelle auftretende Leckblutungen zuverlässig erkannt werden können.

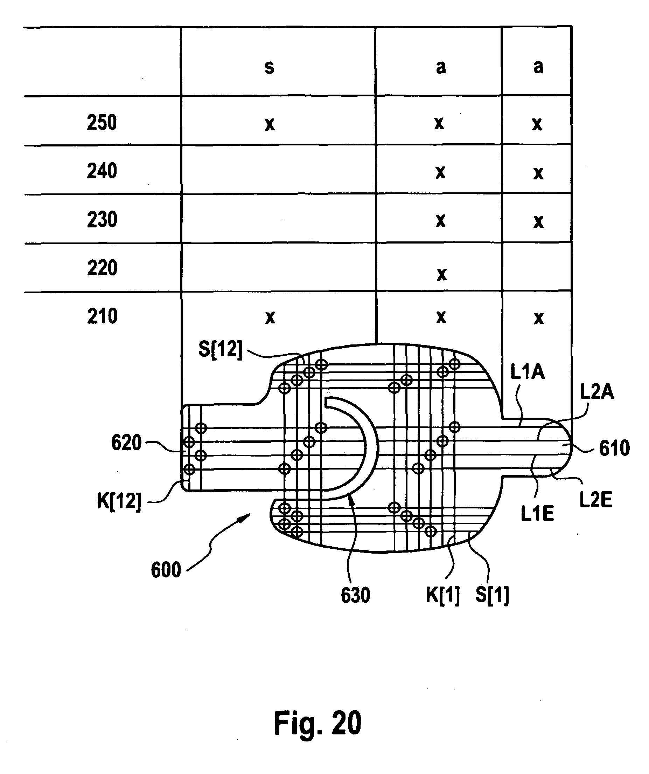

Fig. 20 zeigt eine weitere Ausführungsform des Pads 600, das nicht umgeklappt zu werden braucht, um beidseitig sensitiv zu sein. Die elektrisch leitende Struktur des Pads wird von 12 Kettfäden K [1] bis K [12] und 12 Schussfäden S[1] bis S [12] gebildet. Das Pad 600 weist an der Seite, die der Lasche 610 gegenüberliegt, einen Ansatz 620 auf, der im Bereich der Punktionsstelle zu liegen kommt. Im Bereich dieses Ansatzes 620 ist das Pad an der Unterseite, in den übrigen Bereichen an der Oberseite sensitiv (Tabelle). Ein sich an den Ansatz anschließender halbkreisförmiger schmaler Einschnitt 630 durchtrennt die Fäden wieder so, dass die beiden Stromkreise der elektrisch leitenden Struktur nicht redundant sind.

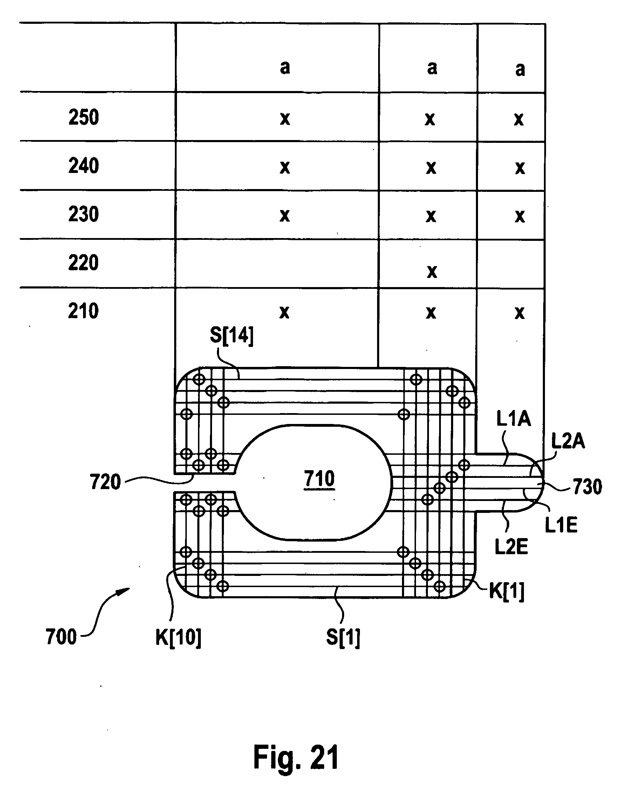

Fig. 21 zeigt eine weitere Ausführungsform eines Pads 700, dass nur an der Oberseite sensitiv ist (Tabelle). Die elektrisch leitende Struktur des Pads wird von 10 Kettfäden K [1] bis K [10] und 14 Schussfäden S[1] bis S [14] gebildet. Das Pad zeichnet sich durch einen zentralen, beispielsweise ovalen Ausschnitt 710 aus, von dem sich ein schmaler Einschnitt 720 bis zu der Seite des Pads erstreckt, die der Lasche 730 mit den Anschlusskontakten L1A, L1E und L2A, L2E gegenüberliegt. In dem zentralen Ausschnitt 710 kommt die Kanüle zu liegen. Da die Kanüle von dem Pad nahezu vollständig umschlossen wird, können Leckblutungen auch in entgegengesetzter Richtung zur Nadel zuverlässig erkannt werden.

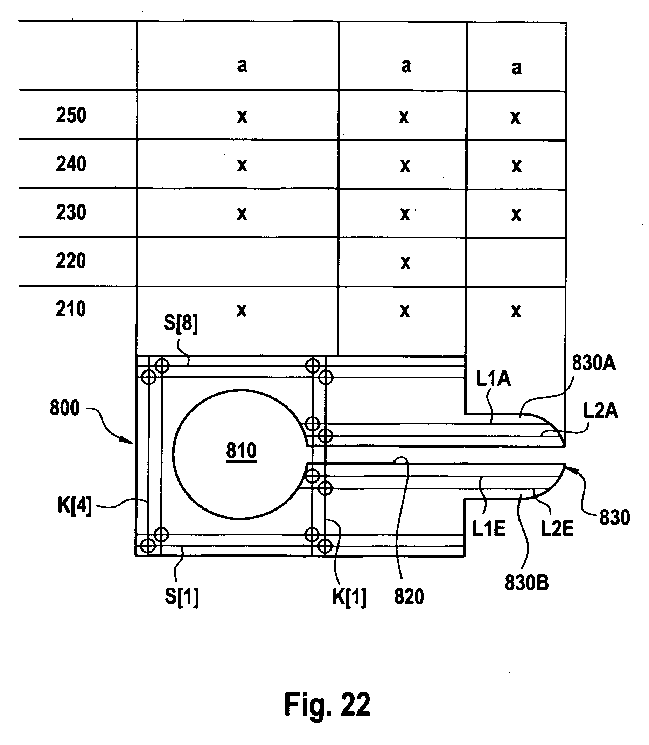

Ein weiteres Ausführungsbeispiel eines nur an der Oberseite sensitiven Pads 800 zeigt Fig. 22. Die elektrisch leitende Struktur des Pads wird von 4 Kettfäden K [1] bis K [4] und 8 Schussfäden S[1] bis S [8] gebildet. Das Pad unterscheidet sich von dem Pad von Fig. 21 im Wesentlichen dadurch, dass sich der von dem zentralen Ausschnitt 810 ausgehende Einschnitt 820 nicht zu der Seite, die der Lasche 830 gegenüberliegt, sondern durch die Lasche selbst erstreckt. Dadurch wird die Lasche 830 in zwei Hälften 830A, 830B geteilt, auf denen jeweils zwei Anschlusskontakte L1A, L1E bzw. L2A, L2E angeordnet sind. Der zentrale Ausschnitt 810 ist bei dieser Ausführungsform nicht oval, sondern kreisförmig.

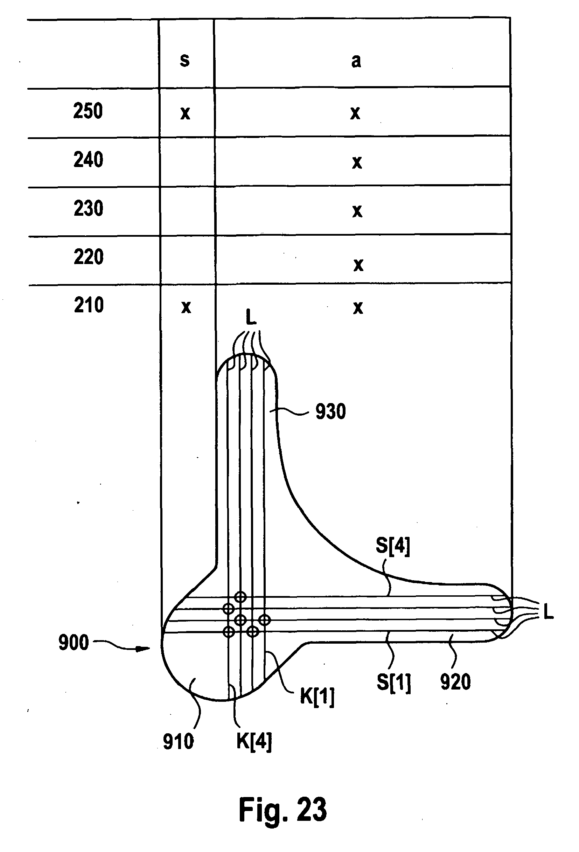

Eine weitere Ausführungsform zeigt Fig. 23, die beidseitig sensitiv ist. Das Pad 900 weist einen zentralen Abschnitt 910 auf, der an der Unterseite sensitiv ist. Mit dem zentralen Abbschnitt 910 wird das Pad auf die Punktionsstelle gelegt. Die elektrisch leitende Struktur des Pads wird von 4 Kettfäden K [1] bis K [4] und 4 Schussfäden S[1] bis S [4] gebildet, die sich in einem zentralen Abschnitt 910 kreuzen. Die Kreuzungspunkte, an denen die Fäden kontaktieren, sind durch Kreise gekennzeichnet.

Von den anderen Pads unterscheidet sich diese Ausführungsform insbesondere dadurch, dass das Pad über zwei Laschen 920, 930 mit jeweils vier Anschlusskontakten L1A, L1E bzw. L2A, L2E verfügt, die an den zentralen Abschnitt 910 angeformt sind. Die beiden Laschen 920, 930 können beispielsweise einen Winkel von 90° einschließen. Der Vorteil dieser Ausführungsform liegt darin, dass das Anschlussteil an zwei verschiedenen Stellen an das Pad angeschlossen werden kann. Bei dieser Ausführungsform ist die Leiterschleife nicht redundant ausgeführt.

## Patentansprüche

1. Vorrichtung zum Detektieren von Feuchtigkeit zur Verwendung mit einer Vorrichtung zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung, bei der Blut eines Patienten über eine arterielle Schlauchleitung, die eine arterielle Kanüle aufweist, von dem Patienten abgeführt wird, und über eine venöse Schlauchleitung, die eine venöse Punktionskanüle aufweist, dem Patienten zugeführt wird,
wobei die Vorrichtung zum Detektieren von Feuchtigkeit als ein auf die Haut des Patienten aufzulegendes Flächengebilde ausgebildet ist, das als Feuchtigkeitssensor eine elektrisch leitende Struktur aufweist,
**dadurch gekennzeichnet, dass**
die Vorrichtung zum Detektieren von Feuchtigkeit als ein Gewebe aus nichtleitfähigen Kettfäden und nicht leitfähigen Schussfäden sowie leitfähigen Kettfäden (50) und leitfähigen Schussfäden (60) ausgebildet ist, wobei die leitfähigen und nicht-leitfähigen Kett- und Schussfäden derart angeordnet sind, dass die elektrisch leitende Struktur ausgebildet ist.

2. Vorrichtung nach Anspruch 1, **dadurch gekennzeichnet, dass** die elektrisch leitende Struktur eine erste Leiterbahn (80) und eine zweite Leiterbahn (90) aufweist, wobei die Enden der beiden Leiterbahnen als Anschlusskontakte (80C, 80D; 90C, 90D) ausgebildet sind und die erste Leiterbahn und die zweite Leiterbahn in einer Mehrzahl von Abschnitten (80A, 80B; 90A, 90B) nebeneinander liegend angeordnet sind.

3. Vorrichtung nach Anspruch 1, **dadurch gekennzeichnet, dass** die elektrisch leitende Struktur eine als geschlossene Leiterschleife ausgebildete Leiterbahn (85) aufweist, deren Enden als Anschlusskontakte (85C, 85D) ausgebildet sind, wobei die Leiterbahn eine Mehrzahl von nebeneinander liegend angeordneten Abschnitten (85A, 85B) aufweist.

4. Vorrichtung nach einem der Ansprüche 1 bis 3, **dadurch gekennzeichnet, dass** sich die elektrisch leitende Struktur aus einer Mehrzahl von in einer ersten Richtung verlaufenden und einer Mehrzahl von in einer zweiten Richtung verlaufenden elektrisch leitfähigen Abschnitten (80A, 80B; 90A, 90B; 85A, 85B) zusammensetzt, wobei die erste und zweite Richtung orthogonal zueinander stehen.

5. Vorrichtung nach einem der Ansprüche 1 bis 4, **dadurch gekennzeichnet, dass** das textile Flächengebilde zumindest teilweise als ein Gewebe mit mehreren Lagen ausgebildet ist.

6. Vorrichtung nach Anspruch 5, **dadurch gekennzeichnet, dass** die elektrisch leitfähigen und elektrisch nicht-leitfähigen Kett- und Schussfäden (50, 60) in dem Mehrlagengewebe derart angeordnet sind, dass
eine auf die Haut des Patienten aufzulegende Lage, die nicht elektrisch leitend ist,
eine Lage, in der die in der ersten Richtung verlaufenden elektrisch leitfähigen Abschnitte der Leiterbahn liegen, und
eine Lage, in der die in der zweiten Richtung verlaufenden elektrisch leitfähigen Abschnitte der Leiterbahn liegen,
ausgebildet ist.

7. Vorrichtung nach Anspruch 6, **dadurch gekennzeichnet, dass** die elektrisch leitfähigen und elektrisch nicht-leitfähigen Kett- und Schussfäden (50, 60) in dem Mehrlagengewebe derart angeordnet sind, dass zwischen der Lage, in der die in der ersten Richtung verlaufenden Abschnitte der Leiterbahn liegen, und der Lage, in der die in der zweiten Richtung verlaufenden Abschnitte der Leiterbahn liegen, eine Zwischenlage liegt, die nicht elektrisch leitend ist.

8. Vorrichtung nach Anspruch 6 oder 7, **dadurch gekennzeichnet, dass** zur Schaffung von elektrischen Kontaktstellen (70) elektrisch leitfähige Kettfäden (50) die Lage in dem Mehrlagengewebe derart partiell wechseln, dass elektrisch leitfähige Kett- und Schussfäden an den Kreuzungspunkten kontaktieren.

9. Vorrichtung nach einem der Ansprüche 2 bis 8, **dadurch gekennzeichnet, dass** die Abschnitte der Leiterbahn von mehreren nebeneinander verlaufenden elektrisch leitfähigen Kettfäden oder Schussfäden (50, 60) gebildet werden.

10. Vorrichtung nach Anspruch 9, **dadurch gekennzeichnet, dass** das textile Flächengebilde derart ausgeschnitten ist, dass ein Teil der nebeneinander verlaufenden elektrisch leitfähigen Kettfäden oder Schussfäden (50, 60) durchtrennt ist.

11. Vorrichtung nach Anspruch 10, **dadurch gekennzeichnet, dass** das textile Flächengebilde einen kreisförmigen Ausschnitt (40G) oder kreuzförmigen Ausschnitt (40F) aufweist.

12. Vorrichtung nach einem der Ansprüche 1 bis 11, **dadurch gekennzeichnet, dass** das textile Flächengebilde U-förmig ausgebildet ist.

13. Vorrichtung nach einem der Ansprüche 1 bis 11, **dadurch gekennzeichnet, dass** das textile Flächengebilde kreisförmig ausgebildet ist.

14. Vorrichtung nach einem der Ansprüche 1 bis 13, **dadurch gekennzeichnet, dass** das textile Flächengebilde eine Lasche (40E) aufweist, an der die Anschlusskontakte (80C, 80D; 90C, 90D) angeordnet sind.

15. Vorrichtung nach einem der Ansprüche 1 bis 14, **dadurch gekennzeichnet, dass** das textile Flächengebilde einen Abschnitt mit einem Ausschnitt (310; 540) und einen Abschnitt mit einer Abdeckung (340; 560) für den Ausschnitt aufweist, wobei die elektrisch leitende Struktur derart ausgebildet ist, dass das textile Flächengebilde an der Oberseite für Feuchtigkeit sensitiv ist.

16. Vorrichtung (B) zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung, bei der Blut eines Patienten über eine arterielle Schlauchleitung, die eine arterielle Kanüle aufweist, von dem Patienten abgeführt wird, und über eine venöse Schlauchleiturig, die eine venöse Punktionskanüle aufweist, dem Patienten zugeführt wird, und mit einer Vorrichtung zum Detektieren von Feuchtigkeit nach einem der Ansprüche 1 bis 15.

17. Vorrichtung nach Anspruch 16, **dadurch gekennzeichnet, dass** die Überwachungsvorrichtung (B) eine an die Vorrichtung zum Detektieren von Feuchtigkeit (40) anschließbare Auswerteinheit (41) aufweist.

18. Vorrichtung nach Anspruch 16 oder 17, **dadurch gekennzeichnet, dass** die Überwachungsvorrichtung einen Anschlussteil (150; 170) aufweist, an den die Vorrichtung (40) zum Detektieren von Feuchtigkeit anschließbar ist.

19. Vorrichtung nach Anspruch 18, **dadurch gekennzeichnet, dass** der Anschlussteil (150; 170) vier Anschlusskontakte (156 -159; 181 - 184) aufweist, wobei zwei Anschlusskontakte (160, 164; 181, 184) an ein Verbindungskabel (42) zur Herstellung einer elektrischen Verbindung zwischen der Auswerteinheit (41) der Überwachungsvorrichtung (B) und der Vorrichtung zum Detektieren von Feuchtigkeit (40) angeschlossen sind , und zwei Anschlusskontakte (162, 163; 182, 183) über einen Abschlusswiderstand (R) elektrisch miteinander verbunden sind.

20. Vorrichtung nach Anspruch 19, **dadurch gekennzeichnet, dass** der Anschlussteil (150; 170) als Klemmvorrichtung zum Verklemmen des textilen Flächengewebes ausgebildet ist.

21. Blutbehandlungsvorrichtung mit einem extrakorporalen Blutkreislauf (I), der eine arterielle Blutleitung (6) mit einer arteriellen Kanüle (5) und eine venöse Blutleitung (7) mit einer venösen Kanüle (8) aufweist, und mit einer Vorrichtung zur Überwachung (B) des arteriellen und/oder venösen Gefäßzugangs nach einem der Ansprüche 16 bis 20.

22. Verfahren zur Herstellung von Vorrichtungen zum Detektieren von Feuchtigkeit zur Verwendung mit einer Vorrichtung zur Überwachung eines Zugangs zu einem Patienten für eine Einrichtung, mit der über eine Schlauchleitung eine Flüssigkeit einem Patienten zugeführt und/oder eine Flüssigkeit von dem Patienten abgeführt wird, insbesondere zur Überwachung des Gefäßzugangs bei einer extrakorporalen Blutbehandlung, bei der Blut eines Patienten über eine arterielle Schlauchleitung, die eine arterielle Kanüle aufweist, von dem Patienten abgeführt wird, und über eine venöse Schlauchleitung, die eine venöse Punktionskanüle aufweist, dem Patienten zugeführt wird, mit folgenden Verfahrenschritten,
Weben eines Gewebes aus nicht-leitfähigen Kettfäden und nicht leitfähigen Schussfäden sowie leitfähigen Kettfäden und leitfähigen Schussfäden, wobei die leitfähigen und nicht-leitfähigen Kett- und Schussfäden derart angeordnet werden, dass die leitfähigen und nicht-leitfähigen Kett- und Schussfäden eine elektrisch leitende Struktur bilden, und
Vereinzeln der einzelnen Vorrichtungen zum Detektieren von Feuchtigkeit.

23. Verfahren nach Anspruch 22, **dadurch gekennzeichnet, dass** die auf die Haut des Patienten aufzulegende Seite des textilen Flächengebildes eine Klebschicht oder eine Klebefolie und auf die Klebeschicht bzw. Klebefolie ein die Klebeschicht bzw. Klebefolie abdeckendes Deckmaterial aufgebracht wird.

24. Verfahren nach Anspruch 23, **dadurch gekennzeichnet, dass** die Klebschicht oder Klebefolie für Feuchtigkeit undurchlässig ist.

## Claims

1. Device for detecting moisture for use with a device for monitoring an access to a patient for an apparatus with which a fluid is supplied to a patient and/or a fluid is carried away from the patient via a hose line, in particular for monitoring the vascular access in an extracorporeal blood treatment, in which blood of a patient is carried away from the patient via an arterial hose line which has an arterial cannula and is supplied back to the patient via a venous hose line which has a venous puncture cannula, the device for detecting moisture being configured as a fabric to be placed onto the skin of the patient, said fabric comprising an electrically conductive structure as a moisture sensor, **characterised in that** the device for detecting moisture is configured as a woven fabric made from non-conductive warp threads, non-conductive weft threads, conductive warp threads (50) and conductive weft threads (60), the conductive and non-conductive warp and weft threads being arranged so as to form the electrically conductive structure.

2. Device according to claim 1, **characterised in that** the electrically conductive structure comprises a first strip conductor (80) and a second strip conductor (90), the ends of the two strip conductors being configured as terminal contacts (80C, 80D; 90C, 90D), and the first strip conductor and the second strip conductor being arranged side by side in a plurality of portions (80A, 80B; 90A, 90B).

3. Device according to claim 1, **characterised in that** the electrically conductive structure comprises a strip conductor (85) configured as a closed conductor loop, the ends of said conductor being configured as terminal contacts (85C, 85D), the strip conductor comprising a plurality of portions (85A, 85B) arranged side by side.

4. Device according to any of claims 1 to 3, **characterised in that** the electrically conductive structure comprises a plurality of electrically conductive portions (80A, 80B; 90A, 90B; 85A, 85B) extending in a first direction and extending in a second direction, the first and second directions being orthogonal to one another.

5. Device according to any of claims 1 to 4, **characterised in that** the textile fabric is configured at least in part as a woven fabric comprising a plurality of layers.

6. Device according to claim 5, **characterised in that** the electrically conductive and electrically non-conductive warp and weft threads (50, 60) are arranged in the multi-layer woven fabric so as to form:
a layer which is to be placed on the skin of the patient and is non-conductive,
a layer in which the electrically conductive portions of the strip conductor extend in the first direction, and
a layer in which the electrically conductive portions of the strip conductor extend in the second direction.

7. Device according to claim 6, **characterised in that** the electrically conductive and electrically non-conductive warp and weft threads (50, 60) are arranged in the multi-layer woven fabric such that an intermediate layer, which is not electrically conductive, is located between the layer in which the portions of the strip conductor extending in the first direction are positioned and the layer in which the portions of the strip conductor extending in the second direction are positioned.

8. Device according to either claim 6 or claim 7, **characterised in that**, in order to create electrical contact points (70), electrically conductive warp threads (50) change position in the multi-layer woven fabric in part, in such a way that electrically conductive warp and weft threads come into contact at the points of intersection.

9. Device according to any of claims 2 to 8, **characterised in that** the portions of the strip conductor are formed by a plurality of electrically conductive warp threads or weft threads (50, 60) extending side by side.

10. Device according to claim 9, **characterised in that** the textile fabric is cut out in such a way that some of the electrically conductive warp threads or weft threads (50, 60) extending side by side are severed.

11. Device according to claim 10, **characterised in that** the textile fabric comprises a circular cutout (40G) or a cross-shaped cutout (40F).

12. Device according to any of claims 1 to 11, **characterised in that** the textile fabric is U-shaped.

13. Device according to any of claims 1 to 11, **characterised in that** the textile fabric is circular.

14. Device according to any of claims 1 to 13, **characterised in that** the textile fabric comprises a tab (40E) on which the terminal contacts (80C, 80D; 90C, 90D) are arranged.

15. Device according to any of claims 1 to 14, **characterised in that** the textile fabric comprises a portion having a cutout (310; 540) and a portion having a cover (340; 560) for the cutout, the electrically conductive structure being configured in such a way that the textile fabric is sensitive to moisture on the upper surface.

16. Device (B) for monitoring an access to a patient for an apparatus with which a fluid is supplied to a patient and/or a fluid is carried away from the patient via a hose line, in particular for monitoring the vascular access in an extracorporeal blood treatment, in which blood of a patient is carried away from the patient via an arterial hose line which has an arterial cannula and is supplied back to the patient via a venous hose line which has a venous puncture cannula, said device comprising a device for detecting moisture according to any of claims 1 to 15.

17. Device according to claim 16, **characterised in that** the monitoring device (B) comprises an evaluation unit (41) which can be connected to the device for detecting moisture (40).

18. Device according to either claim 16 or claim 17, **characterised in that** the monitoring device comprises a connection part (150; 170) to which the device (40) for detecting moisture can be connected.

19. Device according to claim 18, **characterised in that** the connection part (150; 170) comprises four terminal contacts (156 -159; 181 - 184), two terminal contacts (160, 164; 181, 184) being connected to a connection cable (42) in order to produce an electrical connection between the evaluation unit (41) of the monitoring device (B) and the device for detecting moisture (40), and two terminal contacts (162, 163; 182, 183) being electrically interconnected via a terminating resistor (R).

20. Device according to claim 19, **characterised in that** the connection part (150; 170) is configured as a clamping device for clamping the textile fabric.

21. Blood treatment device having an extracorporeal blood circuit (I), the device comprising an arterial blood line (6) having an arterial cannula (5) and a venous blood line (7) having a venous cannula (8), and comprising a device for monitoring (B) the arterial and/or venous vascular access according to any of claims 16 to 20.

22. Method for producing devices for detecting moisture for use with a device for monitoring an access to a patient for an apparatus with which a fluid is supplied to a patient and/or a fluid is carried away from the patient via a hose line, in particular for monitoring the vascular access in an extracorporeal blood treatment, in which blood of a patient is carried away from the patient via an arterial hose line which has an arterial cannula and is supplied back to the patient via a venous hose line which has a venous puncture cannula, said method comprising the method steps of:
weaving a woven fabric made from non-conductive warp threads, non-conductive weft threads, conductive warp threads and conductive weft threads, the conductive and non-conductive warp and weft threads being arranged such that the conductive and non-conductive warp and weft threads form an electrically conductive structure, and
separating the individual devices for detecting moisture.

23. Method according to claim 22, **characterised in that** an adhesive layer or adhesive film is applied to the side of the textile fabric which is to be placed on the skin of the patient, and a covering material covering the adhesive layer or adhesive film is applied to the adhesive layer or adhesive film.

24. Method according to claim 23, **characterised in that** the adhesive layer or adhesive film is impermeable to moisture.

## Revendications

1. Dispositif permettant de détecter l'humidité, destiné à être utilisé avec un dispositif pour la surveillance d'une voie d'entrée sur un patient pour une installation, par laquelle, via une conduite flexible, un liquide est amené vers un patient et/ou un liquide est extrait d'un patient, en particulier pour la surveillance d'une voie d'entrée vasculaire dans le cas d'un traitement du sang extracorporel, pendant lequel le sang d'un patient est extrait du patient via une conduite flexible artérielle qui comporte une canule artérielle et est acheminé vers le patient via une conduite flexible veineuse qui comporte un trocart veineux,
ledit dispositif permettant de détecter l'humidité étant configuré sous la forme d'une nappe destinée à être posée sur la peau du patient et comportant une structure électroconductrice formant un capteur d'humidité,
**caractérisé en ce que**
le dispositif permettant de détecter l'humidité est configuré sous la forme d'un tissu en fils de chaîne non conducteurs et fils de trame non conducteurs, et en fils de chaîne conducteurs (50) et fils de trame conducteurs (60), les fils de chaîne et fils de trame conducteurs et non conducteurs étant agencés de manière à former la structure électroconductrice.

2. Dispositif selon la revendication 1, **caractérisé en ce que** la structure électroconductrice comporte une première piste conductrice (80) et une deuxième piste conductrice (90), les extrémités des deux pistes conductrices étant réalisées sous forme de contacts de connexion (80C, 80D ; 90C, 90D) et la première piste conductrice et la deuxième piste conductrice étant agencées en une pluralité de tronçons (80A, 80B ; 90A, 90B) disposés les uns à côté des autres.

3. Dispositif selon la revendication 1, **caractérisé en ce que** la structure électroconductrice comporte une piste conductrice (85), réalisée sous forme de boucle conductrice fermée, dont les extrémités sont réalisées sous forme de contacts de connexion (85C, 85D), la piste conductrice comportant une pluralité de tronçons (85A, 85B) disposés les uns à côté des autres.

4. Dispositif selon l'une quelconque des revendications 1 à 3, **caractérisé en ce que** la structure électroconductrice est constituée d'une pluralité de tronçons électroconducteurs (80A, 80B ; 90A, 90B ; 85A, 85B) qui s'étendent dans une première direction et une pluralité de tronçons électroconducteurs qui s'étendent dans une deuxième direction, la première et la deuxième direction étant orthogonales l'une à l'autre.

5. Dispositif selon l'une quelconque des revendications 1 à 4, **caractérisé en ce que** la nappe textile est réalisée au moins en partie sous la forme d'un tissu à plusieurs couches.

6. Dispositif selon la revendication 5, **caractérisé en ce que** les fils de chaîne et fils de trame (50, 60) électroconducteurs et non électroconducteurs sont agencés dans le tissu multicouche de manière à constituer
une couche à poser sur la peau du patient, laquelle est non électroconductrice,
une couche, dans laquelle sont disposés les tronçons électroconducteurs de la piste conductrice, orientés dans la première direction, et
une couche, dans laquelle sont disposés les tronçons électroconducteurs de la piste conductrice, orientés dans la deuxième direction.

7. Dispositif selon la revendication 6, **caractérisé en ce que** les fils de chaîne et fils de trame (50, 60) électroconducteurs et non électroconducteurs sont agencés dans le tissu multicouche de telle sorte qu'une couche intermédiaire, qui est non électroconductrice, est disposée entre la couche, dans laquelle sont disposés les tronçons de la piste conductrice orientés dans la première direction, et la couche, dans laquelle sont disposés les tronçons de la piste conductrice orientés dans la deuxième direction.

8. Dispositif selon la revendication 6 ou 7, **caractérisé en ce que** pour créer des points de contact (70) électriques, les fils de chaîne électroconducteurs (50) changent partiellement la couche dans le tissu multicouche de telle sorte que les fils de chaîne et fils de trame électroconducteurs entrent en contact au niveau des points d'intersection.

9. Dispositif selon l'une quelconque des revendications 2 à 8, **caractérisé en ce que** les tronçons de la piste conductrice sont formés par plusieurs fils de chaîne ou fils de trame (50, 60) électroconducteurs s'étendant les uns à côté des autres.

10. Dispositif selon la revendication 9, **caractérisé en ce que** la nappe textile est découpée de telle sorte qu'une partie des fils de chaîne ou fils de trame (50, 60) électroconducteurs, qui s'étendent les uns à côté des autres, est sectionnée.

11. Dispositif selon la revendication 10, **caractérisé en ce que** la nappe textile comporte une découpe circulaire (40G) ou une découpe en croix (40F).

12. Dispositif selon l'une quelconque des revendications 1 à 11, **caractérisé en ce que** la nappe textile est réalisée en forme de U.

13. Dispositif selon l'une quelconque des revendications 1 à 11, **caractérisé en ce que** la nappe textile est circulaire.

14. Dispositif selon l'une quelconque des revendications 1 à 13, **caractérisé en ce que** la nappe textile comporte une patte (40E) sur laquelle sont disposés les contacts de connexion (80C, 80D ; 90C, 90D).

15. Dispositif selon l'une quelconque des revendications 1 à 14, **caractérisé en ce que** la nappe textile comporte une partie avec une découpe (310 ; 540) et une partie avec un recouvrement (340 ; 560) pour la découpe, la structure électroconductrice étant configurée de telle sorte que la nappe textile est sensible à l'humidité sur la face supérieure.

16. Dispositif (B) permettant de surveiller une voie d'entrée sur un patient pour une installation, par laquelle, via une conduite flexible, un liquide est amené vers un patient et/ou un liquide est extrait d'un patient, en particulier pour la surveillance d'une voie d'entrée vasculaire dans le cas d'un traitement du sang extracorporel, pendant lequel le sang d'un patient est extrait du patient via une conduite flexible artérielle qui comporte une canule artérielle et est acheminé vers le patient via une conduite flexible veineuse qui comporte un trocart veineux, et avec un dispositif de détection de l'humidité selon l'une quelconque des revendications 1 à 15.

17. Dispositif selon la revendication 16, **caractérisé en ce que** le dispositif de surveillance (B) comporte une unité d'analyse (41) pouvant être raccordée au dispositif de détection de l'humidité (40).

18. Dispositif selon la revendication 16 ou 17, **caractérisé en ce que** le dispositif de surveillance comporte une partie de raccordement (150 ; 170) à laquelle peut être raccordé le dispositif de détection de l'humidité (40).

19. Dispositif selon la revendication 18, **caractérisé en ce que** la partie de raccordement (150 ; 170) comporte quatre contacts de connexion (156 - 159 ; 181 - 184), deux contacts de connexion (160, 164 ; 181, 184) étant connectés à un câble de liaison (42) destiné à établir une liaison électrique entre l'unité d'analyse (41) du dispositif de surveillance (B) et le dispositif de détection de l'humidité (40), et deux contacts de connexion (162, 163 ; 182, 183) étant reliés électriquement entre eux via une résistance de terminaison (R).

20. Dispositif selon la revendication 19, **caractérisé en ce que** la partie de raccordement (150 ; 170) est réalisée sous la forme d'un dispositif de serrage pour bloquer la nappe textile.

21. Dispositif de traitement du sang comportant un circuit extracorporel (I), qui comporte une ligne artérielle (6) avec une canule artérielle (5) et une ligne veineuse (7) avec une canule veineuse (8), et comportant un dispositif de surveillance (B) de la voie d'abord vasculaire artérielle et/ou veineuse selon l'une quelconque des revendications 16 à 20.

22. Procédé destiné à la réalisation de dispositifs de détection de l'humidité, destinés à être utilisés avec un dispositif pour la surveillance d'une voie d'entrée sur un patient pour une installation, par laquelle, via une conduite flexible, un liquide est amené vers un patient et/ou un liquide est extrait d'un patient, en particulier pour la surveillance d'une voie d'entrée vasculaire dans le cas d'un traitement du sang extracorporel, pendant lequel le sang d'un patient est extrait du patient via une conduite flexible artérielle qui comporte une canule artérielle et est acheminé vers le patient via une conduite flexible veineuse qui comporte un trocart veineux, comportant les étapes suivantes :
tissage d'un tissu en fils de chaîne non conducteurs et fils de trame non conducteurs, ainsi qu'en fils de chaîne conducteurs et fils de trame conducteurs, les fils de chaîne et fils de trame conducteurs et non conducteurs étant agencés de manière à former une structure électroconductrice, et
séparation des différents dispositifs permettant de détecter l'humidité.

23. Procédé selon la revendication 22, **caractérisé en ce que** sur la face de la nappe textile destinée à être posée sur la peau du patient est appliquée une couche adhésive ou un film adhésif, et sur la couche adhésive ou le film adhésif est appliqué un matériau de protection recouvrant la couche adhésive ou le film adhésif.

24. Procédé selon la revendication 23, **caractérisé en ce que** la couche adhésive ou le film adhésif sont imperméables à l'humidité.
